(19) Europäisches Patentamt European Patent Office Office européen des brevets

(11) **EP 4 512 895 A1**

(12) **EUROPEAN PATENT APPLICATION**

(43) Date of publication:
**26.02.2025 Bulletin 2025/09**

(21) Application number: **23192550.4**

(22) Date of filing: **22.08.2023**

(51) International Patent Classification (IPC):
**C12N 9/42** (2006.01)

(52) Cooperative Patent Classification (CPC):
**C12Y 302/0108; C12N 9/2402;** C07K 2319/02;
C07K 2319/20; C07K 2319/35; C07K 2319/50

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **AB Enzymes Finland Oy
05200 Rajamäki (FI)**

(72) Inventors:
• **MÄKINEN, Susanna
05200 Rajamäki (FI)**
• **JUNTUNEN, Kari
05200 Rajamäki (FI)**
• **SEEFRIED, Michael
64293 Darmstadt (DE)**
• **PURANEN, Terhi
05200 Rajamäki (FI)**
• **ALAPURANEN, Marika
05200 Rajamäki (FI)**

(74) Representative: **Espatent Oy
Kaivokatu 10 D
00100 Helsinki (FI)**

Remarks:
The complete document including Reference
Table(s) and the Sequence Listing(s) can be
downloaded from the EPO website

(54) **FRUCTANASE VARIANTS**

(57)     A variant polypeptide of fructanase, a fusion protein and an enzyme composition comprising said variant polypeptide, recombinant host cell producing the variant polypeptide, method for manufacturing the variant polypeptide, a use of the variant polypeptide to degrade and modify fructan containing material, and a premix for baking comprising the variant polypeptide.

**Fig. 1**

EP 4 512 895 A1

## Description

### TECHNICAL FIELD

The present disclosure generally relates to the field of enzyme technology. The disclosure relates particularly, though not exclusively, to fructanase variants having an improved productivity and/or fructan degradation activity. The present fructanase variants are useful in various applications where degradation of fructan is desired, such as in plant-based and baked products.

### BACKGROUND

This section illustrates useful background information without admission of any technique described herein representative of the state of the art.

Fructans are comprised of fructose residues, normally with a terminal sucrose unit (i.e., a glucose-fructose disaccharide). The linkage position of the fructose residues determines the type of the fructan. The basic types of single-linkage fructans are inulin (fructosyl units linked by β-2,1-linkages) and levan (or phlein, both linked by β-2,6-linkages). Additionally, there exists a mixed-linkage fructan called graminan (containing both β-2,1-linkages and β-2,6-linkages). Common sources of fructans include plants, such as cereals, vegetables, fruits and nuts.

Several fructan degrading enzymes i.e., fructanases, are known in the art. Fructanases are fructosidases or exo-beta-D-fructosidases, which are enzymes that catalyze the hydrolysis of a fructan. More specifically, fructanase catalyzes the hydrolysis of terminal, non-reducing (2->1)- and (2->6)-linked beta-D-fructofuranose residues in fructans. Substrates of fructanase include fructans such as inulin and levan and sucrose.

The use of fructanases for degradation of fructans in plant-based materials have been described for various purposes. Fructanases can be added to hydrolyze fructans, for example, to achieve increased softness of the food, or to bring additional sweetness to the food. In WO2017220864 fructanases are used to degrade fructans, to obtain foodstuff suitable for a diet low in fermentable carbohydrates, known as FODMAP.

However, fructanase enzymes are often produced in insufficient quantities, the production yields thereby not being economically feasible. Moreover, the activity of the known fructanase enzymes may be low and inadequate to be used, for example, with foodstuff, to hydrolyze fructans efficiently. Therefore, new fructanase enzymes are needed, having higher production level and specific fructan degrading activity.

It is an object of the present disclosure to provide fructanase variants that exhibit fructanase activity and that have improved properties that allow their use in industrial processes. Yet another object of the present disclosure is to provide fructanase variants that can be used in enzyme compositions and in foodstuff for fructan degradation.

### SUMMARY

The appended claims define the scope of protection. Any examples and technical descriptions of apparatuses, products and/or methods in the description and/or drawings not covered by the claims are presented not as embodiments of the invention but as background art or examples useful for understanding the invention.

The present disclosure provides a novel variant polypeptide with fructanase activity, being able to efficiently degrade fructan. The disclosure relates particularly, though not exclusively, to fructanase variants having an increased activity and/or productivity.

According to a first aspect there is provided a variant polypeptide having:

fructanase activity;
an amino acid sequence having at least 80 % amino acid sequence identity with the amino acids 383 - 858 of SEQ ID NO: 1; and
a size of 476 - 1165 amino acids;

wherein the amino acid numbering of the variant polypeptide corresponds to the amino acid numbering of the SEQ ID NO: 1.

It was surprisingly found that production of a fructanase polypeptide GH32 catalytic domain without one or more of its other native domain sequences, significantly increases its productivity in production hosts without affecting negatively in its ability to degrade fructans.

More particularly, it was surprisingly found that by providing the variant with the amino acid sequence having at least 80 % amino acid sequence identity with the amino acids 383 - 858 of SEQ ID NO: 1, which variant has the size of 476 - 1165 amino acids, at least the productivity of the variant polypeptide in production hosts is significantly increased.

According to a second aspect there is provided a fusion protein comprising the variant polypeptide according to the first aspect, and at least one:

> amino acid sequence providing a secretory signal sequence;
> an amino acid sequence which facilitates purification, such as an affinity tag or His-tag;
> an amino acid sequence controlling and enhancing the production of the fusion protein, such as a carrier polypeptide;
> a linker sequence;
> an amino acid sequence encoding a protease cleavage site;
> an amino acid sequence having an enzyme activity; and/or
> an amino acid sequence providing for the fusion protein with binding affinity, such as a carbohydrate binding moiety.

According to a third aspect there is provided an enzyme composition comprising the variant polypeptide of the first aspect or the fusion protein of the second aspect.

According to a fourth aspect there is provided a recombinant host cell comprising genetic elements that allow producing at least one variant polypeptide of the first aspect, or the fusion protein of the second aspect.

According to a fifth aspect there is provided a method of manufacturing the variant polypeptide of the first aspect, or the fusion protein of the second aspect, comprising:

cultivating the recombinant host cell of the fourth aspect in conditions allowing production of the at least one variant polypeptide of the first aspect, or the fusion protein of the seconds aspect; and optionally recovering the variant polypeptide or the fusion protein.

According to a sixth aspect there is provided a use of the variant polypeptide of the first aspect, and/or the fusion protein of the second aspect and/or the enzyme composition of the third aspect, for degradation of fructan in plant-based material.

According to a seventh aspect there is provided a premix for baking, comprising the variant polypeptide of the first aspect, and/or the fusion protein of the second aspect and/or the enzyme composition of the third aspect.

According to an eighth aspect there is provided a variant polypeptide having:

> fructanase activity;
> an amino acid sequence having at least 80 % amino acid sequence identity with the amino acids 1-1165 of SEQ ID NO: 1; and
> at least one SLAP domain deleted or at least one non-functional SLAP domain, obtained via deletion or substitution of amino acids,

wherein the amino acid numbering of the variant polypeptide corresponds to the amino acid numbering of the SEQ ID NO: 1.

It is to be understood, that the characterization of the variant polypeptide according to the first aspect applies to the variant polypeptide of the eight aspect, and vice versa. Therefore, in some embodiments, the variant polypeptide according to the first aspect is the variant polypeptide according to the eighth aspect, or the variant polypeptide according to the eighth aspect is the variant polypeptide according to the first aspect.

It was surprisingly found that production of the variant polypeptide comprising fructanase polypeptide catalytic domain and one or more native non-catalytic domain sequences, wherein at least one SLAP domain is deleted, significantly increases the productivity of the variant polypeptide in production hosts without negatively affecting its ability to degrade fructans. It was further surprisingly found that production of the variant polypeptide comprising a fructanase polypeptide catalytic domain and one or more non-functional native SLAP domain sequences, significantly increases the productivity of the variant polypeptide in production hosts without negatively affecting its ability to degrade fructans. More particularly, it was surprisingly found that by providing the variant with the amino acid sequence having at least 80 % amino acid sequence identity with the amino acids 1-1165 of SEQ ID NO: 1, which variant has at least one SLAP domain deleted or at least one non-functional SLAP domain, at least the productivity of the variant polypeptide in production hosts is significantly increased. Thus, production of a variant polypeptide comprising fructanase polypeptide catalytic domain and a subset of non-catalytic native domain sequences significantly increases the productivity of the variant polypeptide in production hosts without negatively affecting its ability to degrade fructans.

The present fructanase variants are useful in various applications where modification, degradation, or partial degradation of fructan is desired, such as in plant and grain-based products, particularly baked products.

Different non-binding example aspects and embodiments have been illustrated in the foregoing. The embodiments in the foregoing are used merely to explain selected aspects or steps that may be utilized in different implementations. Some embodiments may be presented only with reference to certain example aspects. It should be appreciated that corresponding embodiments may apply to other example aspects as well.

**BRIEF DESCRIPTION OF THE FIGURES**

Some example embodiments will be described with reference to the accompanying figures, in which:

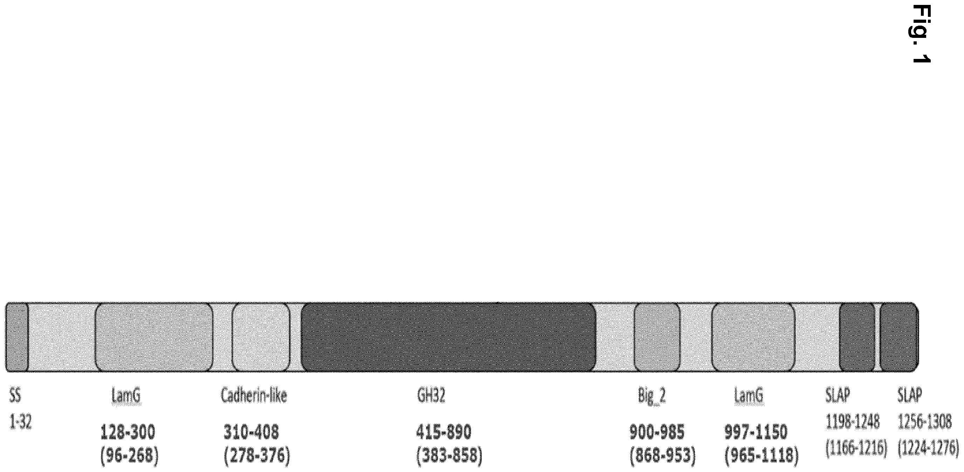

Fig. 1 schematically shows a picture of the *Lactobacillus crispatus* LFRU fructanase with multiple putative domains depicted, according to an example embodiment. The amino acid numbering in parenthesis indicates the amino acid numbering of the different domains of the wild-type *Lactobacillus crispatus* LFRU fructanase without the signal sequence (SS) according to SEQ ID NO:1. The numbers without parentheses indicate the amino acid numbering of the different domains of the same LFRU fructanase including the signal sequence amino acids 1-32. The domains comprised by the wild-type *Lactobacillus crispatus* LFRU fructanase include signal sequence, two Lam G, one Cadherine-like, one GH32, one Big 2, and two SLAP domains.

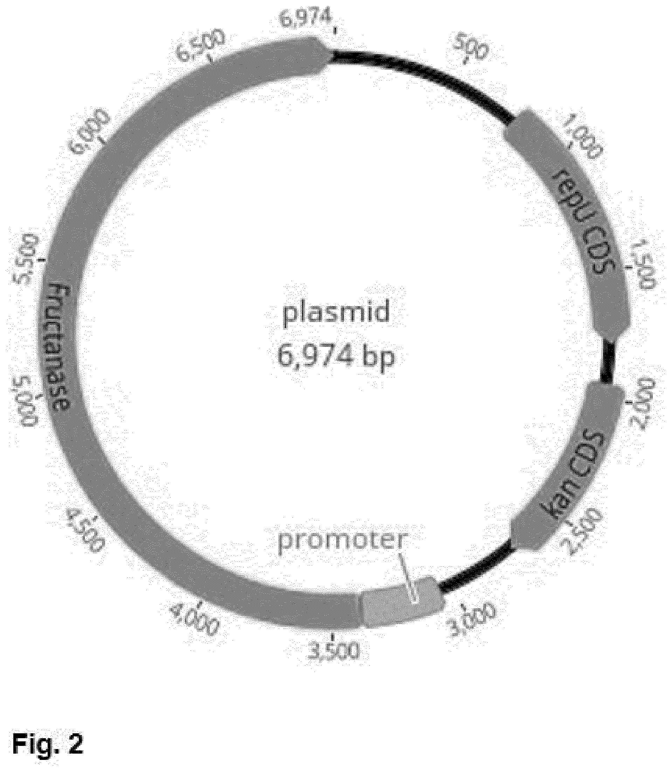

Fig. 2 shows a schematic picture of the expression plasmid used for expression of *Lactobacillus crispatus* LFRU fructanase (wildtype and truncated) genes in *Bacillus subtilis.* Expression of the recombinant genes in the host cell was controlled using *Bacillus amyloliquefaciens* amylase promoter for transcription initiation. The vector backbone is a pUB110 derivate including genes for replication initiator protein (repU CDS) and kanamycin (kan CDS) marker. Picture was generated using Geneious Prime 2021 created by Biomatters.

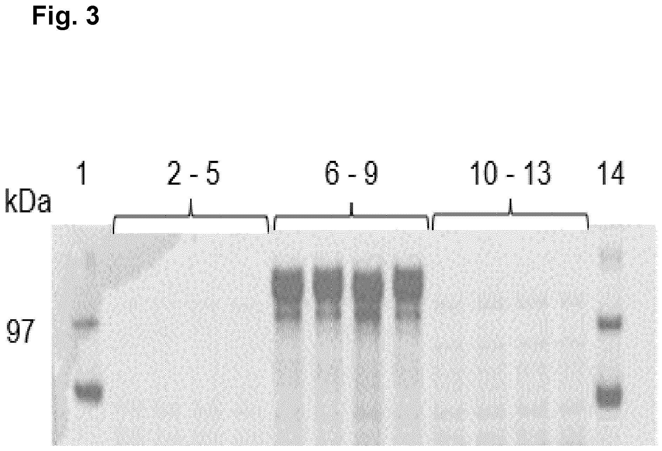

Fig. 3 shows an SDS-PAGE gel prepared with samples of deep-well cultivation supernatants of *Bacillus subtilis* cell strains producing a *Lactobacillus crispatus* truncated LFRU fructanase polypeptide according to SEQ ID NO:2 (replicates included in the lanes 6-9) (sample name "Bs TR" as indicated in Table 2) or wildtype LFRU fructanase polypeptide according to SEQ ID NO:1 (replicates included in the lanes 10-13) (sample name "Bs WT" as indicated in the table 2). Lanes 2-5 are prepared with samples from a host cell strain without any LFRU fructanase production (sample name "Host" as indicated in the table 2). Lanes 1 and 14 contain a standard SDS-PAGE molecular weight marker (Low molecular Weight ladder from Amersham Pharmacia Biotech).

**SEQUENCE LISTING**

| | |
|---|---|
| SEQ ID NO: 1 | Amino acid sequence of a full-length wild-type *Lactobacillus crispatus* LFRU fructanase without a signal peptide, contains the amino acids 1-1279. |
| SEQ ID NO: 2 | Amino acid sequence of a truncated *Lactobacillus crispatus* LFRU fructanase without a S-layer domain and without a signal peptide, contains the amino acids 1-1165 of the full-length wild-type fructosidase. |
| SEQ ID NO: 3 | Sequence of the oligonucleotide primer Vector_fw. |
| SEQ ID NO: 4 | Sequence of the oligonucleotide primer Vector_rev. |
| SEQ ID NO: 5 | Sequence of the oligonucleotide primer Fru_fw1. |
| SEQ ID NO: 6 | Sequence of the oligonucleotide primer Fru_rev1 |
| SEQ ID NO: 7 | Sequence of the oligonucleotide primer Fru_rev2 |

**DETAILED DESCRIPTION**

In the following description, like reference signs denote like elements or steps.

As used herein, the term "fructanase" or "fructosidase" or "exo-beta-D-fructosidase" refers to fructan beta-fructosidase, which is an enzyme with systematic name beta-D-fructan fructohydrolase (EC 3.2.1.80). Fructanase catalyzes the hydrolysis of terminal, non-reducing (2->1)- and (2->6)-linked beta-D-fructofuranose residues in fructans. Substrates of fructanase include inulin, levan and sucrose. Fructanase degrades levans and inulins to fructose and also cleaves sucrose into glucose and fructose and can therefore function as an extracellular invertase.

As used herein, the term "variant polypeptide" refers to a variant which is a polypeptide. The term "variant polypeptide" used herein may refer to the variant polypeptide of the first aspect, the variant polypeptide of the eighth aspect, or to another variant polypeptide. As used herein, the term "variant" means a sequence or a fragment of a sequence (nucleotide or amino acid) inserted, substituted, or deleted by one or more nucleotides/amino acids, or which is chemically modified. The term variant may in some embodiments also include the variant polypeptide (of fructanase), and/or the fusion protein including the variant polypeptide (of fructanase).

As used herein, the term "fructanase variant" and "variant fructanase" and "variant of fructanase" refers to a fructanase molecule obtained by site-directed or random mutagenesis, insertion, substitution, deletion, recombination and/or any other suitable protein engineering method, and which leads into a genetically modified fructanase variant that differs in its amino acid sequence from the parent molecule/ fructanase such as a wild type fructanase. The terms "wild type fructanase", "wild type enzyme", "wild type", or "wt" in accordance with the disclosure, describe a fructanase enzyme with an amino acid sequence found in nature or a fragment thereof. The variant encoding gene can be synthesized, or the parent gene be modified using genetic methods, e.g., by site directed mutagenesis, a technique in which one or more than one mutation are introduced at one or more defined sites in a polynucleotide encoding the parent polypeptide.

The term "parent fructanase polypeptide" or "wild type parent fructanase" or "wild type parent polypeptide" means a fructanase polypeptide, from which a specific variant fructanase polypeptide is derived from.

As used herein, the term "GH32" or the "glycosyl hydrolase 32" refers to a family of enzymes that hydrolyze fructose-containing polysaccharides, comprising at least inulinases, exo-inulinases, levanases, 2,6-β-fructan 6-levanbiohydrolases, fructan β-(2,1)-fructosidases and fructan β-(2,6)-fructosidases.

The term "GH32 domain" refers to the catalytic domain of the glycosyl hydrolase 32 enzymes. For estimating the amino acid region comprising the GH32 domain for each fructanase polypeptide, the ColabFold web tool can be used. For example, the ColabFold web tool was used to predict the structure of *Lactobacillus crispatus* fructosidase SEQ ID NO: 1. and based on the obtained results the catalytic domain GH32 of the SEQ ID NO: 1 comprises the amino acids 383 - 858.

ColabFold is a web-based implementation of the AlphaFold2 protein folding algorithm, capable of predicting the 3D structure of a protein from its amino acid sequence (Mirdita et al., 2022). ColabFold runs on Google Colab, a cloud-based platform for running Jupyter notebooks. Instructions provided in the notebook are used to upload protein sequence in FASTA format. The ColabFold tool performs the following steps: 1) Pre-processing: The input sequence is checked for errors and inconsistencies. 2) Fold recognition: ColabFold first searches a database of known protein structures to identify proteins with similar sequences to input sequence. This information found in the search is used to guide the subsequent structure prediction process. 3) Structure prediction: Using the AlphaFold2 algorithm, ColabFold predicts the 3D structure of protein. The predicted structure is output in PDB format. ColabFold website: (hftps:Hcolab. research. google. com/github/sokrypton/ColabFold/blob/main/AlphaFold2. ipyn b).

As used herein, a "peptide" and a "polypeptide" are amino acid sequences including a plurality of consecutive polymerized amino acid residues. For purpose of this disclosure, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues.

As used herein, the term "mature polypeptide" means any polypeptide wherein at least one signal sequence or signal peptide or signal peptide and a putative pro-peptide, or a carrier peptide or a fusion partner is cleaved off. For example, the "mature polypeptide" of the truncated variant polypeptide according to SEQ ID NO: 2 comprises the amino acids 1-1165.

The term "secretory signal sequence" or "signal sequence" or a "secretory peptide" refers to an amino acid sequence which is a component or a part of a larger polypeptide, and which directs the larger polypeptide through a secretory pathway of a host cell in which it is produced. The secretory signal sequence can be native, or it can be obtained from another source. Depending on the host cell, the larger polypeptide may be cleaved from the secretory peptide during transit through the secretory pathway, thereby forming a mature polypeptide lacking the secretory peptide.

As used herein, a "protein" may refer to a peptide or a polypeptide of any size. A protein may be an enzyme, a protein, an antibody, a membrane protein, a peptide hormone, regulator, or any other protein.

As used herein, "substitution" or "amino acid substitution" means an amino acid residue replacement with an amino acid residue that is different than the original amino acid in that specific replacement position. The term "amino acid substitution" can refer to both, conservative amino acid substitutions and non-conservative amino acid substitutions, which means the amino acid residue is replaced with an amino acid residue having a similar side chain (conservative), or a different side chain (non-conservative), as the original amino acid residue in that place. Substitutions are described using the following nomenclature: amino acid residue in the protein scaffold; position; substituted amino acid residue(s).

As used herein, a "host cell" means any cell type that is susceptible to transformation, transfection, transduction, mating, crossing, CRISPR-Cas or the like with a nucleic acid construct or expression vector comprising a polynucleotide. The term "host cell" encompasses any progeny that is not identical due to mutations that occur during replication. Non-limiting examples of a host cell are fungal cells, preferably a filamentous fungal cell (e.g. *Trichoderma* or *Trichoderma reesei, Aspergillus* or *Aspergillus oryzae* or A. *niger, Thermothelomyces* or *Thermothelomyces heterothallica, Myceliophthora* or *Myceliophthora thermophila, Humicola* or *Humicola insolens, Fusarium* or *Fusarium venenatum),* more preferably bacterial cells, preferably Gram-positive Bacilli (e.g., *Bacillus subtilis, Bacillus pumilus, Alkalihalobacillus alcalophilus, Bacillus amyloliquefaciens, Bacillus velezensis, Brevibacillus brevis, Niallia circulans, Shouchella clausii, Weizmannia coagulans, Halalkalibacterium halodurans, Paenibacillus lautus, Lederbergia lenta, Bacillus licheniformis, Bacillus paralicheniformis Priestia megaterium, Geobacillus stearothermophilus, or Bacillus thuringiensis),* Gram-negative bacteria (e.g., *Escherichia coli*), Actinomycetales (e.g., *Streptomyces* sp., *Nonomuraea flexuosa*) and yeasts (e.g.,

*Saccharomyces cerevisiae, Schizosaccharomyces pombe, Pichia pastoris, Yarrowia lipolytica*). Most preferably, the host cell is a cell of B. *subtilis or B. pumilus.*

As used herein, "sequence identity" means the percentage of exact matches of nucleotides or amino acid residues between two optimally aligned sequences over the number of positions where there are nucleotides/residues present in both sequences. When one sequence has a residue with no corresponding residue in the other sequence, the alignment program allows a gap in the alignment, and that position is not counted in the denominator of the identity calculation. For purposes of the present invention, the sequence identity between two amino acid sequences can be determined using the Needleman-Wunsch algorithm (Needleman et al. 1970) as implemented in the Needle program of the EMBOSS package (Rice et al., 2000), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment}).$$

As used herein, "sequence alignment" of the amino acid sequences means, aligning the sequences using Clustal Omega (1.2.4) multiple sequence alignment program (https://www.ebi.ac.uk/Tools/msa/clustalo/) as described by Sievers et al. 2011, and using the default settings.

As used herein, the term "corresponding positions" or "corresponding amino acid position" or "corresponding amino acids" means aligning at least two amino acid sequences according to identified regions of similarity or identity as pairwise alignment or as multiple sequence alignment, thereby pairing up the corresponding amino acids. The corresponding amino acids between two amino acid sequences need not, but can be, the same amino acids.

Unless otherwise specified, all references to a certain amino acid position refer to an amino acid of the SEQ ID NO: 1 in said position, or to an amino acid present or missing in the corresponding position of an amino acid sequence aligned with SEQ ID NO: 1.

As used herein, the term "fructanase productivity" refers to a level of fructanase production by means of transcription, translation, post-translational modifications and ultimately intracellular or extracellular targeting and/or secretion of the fructanase. Fructanase productivity can thereby refer to the intracellular level of translated fructanase and/or indicate the level of extracellularly secreted fructanase. Methods for protein production by recombinant technology in different host systems are known in the art. Preferably, the fructanase variants are produced as extracellular proteins that are secreted into the culture medium wherein the host cell is cultured, and from which they can be easily recovered and isolated.

As used herein, with "fructanase activity" or "fructan degrading performance" is meant the ability of fructanase to catalyze the hydrolysis of terminal, non-reducing (2->1)- and (2->6)-linked beta-D-fructofuranose residues into fructans. The fructanase activity can be indicated as relative fructanase activity, wherein fructanase activity of a sample is indicated as fold change in relation to the control sample. Example 3 provides an example of a method for determining fructanase activity. The fructanase activity can also be indicated in enzymatic units/ml (U/ml), wherein one enzymatic unit is defined as the amount of the enzyme which produces 1 $\mu$mol of reducing sugars per ml.

As used herein, the term S-layer associated protein" or "SLAP", or "S-layer" domain refers to amino acid sequence homologous to short domain which is found in a variety of bacterial cell surface proteins. As used herein, the term "SLAP domain" can in specific embodiments refer to a SLAP domain belonging to a Pfam family PF03217, i.e., a SLAP domain according to EMBL-EBI PFAM 35.0 database and hidden Markov model profiles (HMM profiles).

As used herein, the "PFAM database", such as PFAM 35.0 database, refers to a database of protein families that includes their annotations and multiple sequence alignments generated using hidden Markov models. In this context is meant the version PFAM 35.0 from November 2021 of the PFAM database, available for all computer platforms from e.g. https://pfam.xfam.org/.

As used herein, the term "HMM profile" refers to hidden Markov model profiles, which are computational algorithms/models used for predicting protein structure and function, which identify significant protein sequence similarities allowing the detection of homologs. HMM profiles also allow encapsulating the evolutionary changes that have occurred in a set of related sequences (i.e., a multiple sequence alignment). HMM profiles capture position-specific information about how conserved each amino acid is in each column of the alignment. HMM profiles may be generated online, and the HMM profiles may be obtained from the PFAM database. HMM profiles may also be generated locally on a computer using the HMMER software package (current version HMMER 3.3.2 from December 2021). HMMER database is available for all computer platforms from http://h m mer. org/. https://pfam.xfam.org/.

As used herein, the term "C-terminal" or "carboxy-terminal" refers to the end of an amino acid chain (protein, polypeptide, peptide), terminated by a free carboxyl group -COOH. With the term "C-terminal amino acid sequence" is meant an amino acid sequence from which the majority of is located at the C-terminal half of the entire amino acid sequence in question.

As used herein, the term "N-terminal" or "amino-terminal" refers to the end of an amino acid chain (protein, polypeptide, or peptide), starting with a free amine group (-NH$_2$). With the term "N-terminal amino acid sequence" is meant an amino acid sequence from which the majority of is located at the N-terminal half of the entire amino acid sequence in question.

As used herein, the term "plant-based material" refers to materials and products that are partially or wholly derived from plants.

With the term "enzyme composition" is meant an enzymatic fermentation product, usually cell-free and possibly isolated and purified, typically produced by a pure culture of a microorganism. The enzyme composition usually comprises a number of different enzymatic activities produced by the microorganism. In another embodiment the enzyme composition is a mixture of monocomponent enzymes, preferably enzymes derived from bacterial or fungal species by using conventional recombinant production techniques. The enzyme composition may contain, for example, stabilators and preservatives which prevent microbial growth and improve storage stability. In an embodiment the term "enzyme composition" means a composition obtained by steps of culturing a host cell and either recovering at least one enzyme, which may be a polypeptide, from the cells or separating the cells from the spent culture medium and obtaining the supernatant comprising the at least one enzyme. The spent culture medium of the production host can be used as such, or the host cells may be removed, and/or it may be concentrated, filtrated or fractionated. It may also be dried. The obtained enzyme composition may be in the form of liquid, flour, powder, granulate or tablets. The enzyme may be in immobilized form in the composition. The enzyme composition may be a monocomponent, or have other enzymes produced by the production host, or a mixture of enzymes from different sources.

The term "carrier" or "carrier polypeptide" refers to a polypeptide into which the protein of interest (fructanase) is translationally fused to improve the yield. The carrier can be either homologous or heterologous to the production host in its origin and can be a full-length protein or a fragment of a protein (e.g., a core, a CBM or a CBM and linker region). It is preferably encoded by a gene or a nucleotide sequence with good expression level.

As used herein, the term "expression" includes any step or all steps involved in the production of a polypeptide in a host cell including, but not limited to, transcription, translation, post-translational modification, and secretion. Expression may be followed by harvesting, i.e., recovering, the host cells or the expressed product.

As used herein, the term "comprising" includes the broader meanings of "including", "containing", and "comprehending", as well as the narrower expressions "consisting of" and "consisting only of".

In an embodiment, the variant polypeptide has: fructanase activity; an amino acid sequence having at least 80 % amino acid sequence identity with the amino acids 383 - 858 of SEQ ID NO: 1; and a size of 476 - 1165 amino acids; wherein the amino acid numbering of the variant polypeptide corresponds to the amino acid numbering of the SEQ ID NO: 1.

In an embodiment, the amino acid numbering of the variant polypeptide corresponds to the amino acid numbering of the SEQ ID NO: 1. SEQ ID NO:1 is LFRU, indicating a full-length *Lactobacillus crispatus* fructosidase amino acid sequence without a signal peptide, comprising 1279 amino acids.

In an embodiment, the variant polypeptide is an extracellular fructosidase and a variant of member of glycosyl hydrolase family 32 (GH32), and the variant polypeptide has fructanase activity. The glycosyl hydrolase 32 (GH32) is a family of enzymes that hydrolyze fructose-containing polysaccharides, comprising at least inulinases, exo-inulinases, levanases, 2,6-β-fructan 6-levanbiohydrolases, fructan β-(2,1)-fructosidases and fructan β-(2,6)-fructosidases. The catalytic domain GH32 of the SEQ ID NO: 1 comprises the amino acids 383-858. In an embodiment, the variant polypeptide has an amino acid sequence having a high, such as 80 % - 100 %, sequence identity with the amino acids comprised by the GH32 domain of the SEQ ID NO: 1. In an embodiment, the amino acid sequence of the variant polypeptide has at least 80 %, but less than 100 % amino acid sequence identity with amino acids 383-858 of SEQ ID NO: 1. In some other embodiments, the amino acid of the variant polypeptide has 100 % amino acid sequence identity with amino acids 383 - 858 of SEQ ID NO: 1. In an embodiment, the variant polypeptide is a GH32 family fructanase which may have endo and/or exo activity.

In an embodiment, the amino acid sequence of the variant polypeptide has at least 80 % amino acid sequence identity with amino acids 383 - 858 of SEQ ID NO: 1 and a size of 476 - 1165 amino acids.

In an embodiment, the variant polypeptide has amino acids corresponding to amino acids 1 - 1165 of the SEQ ID NO: 1 or less, preferably the variant polypeptide has amino acids corresponding to amino acids 1 - 858 of the SEQ ID NO: 1 or less, more preferably the variant polypeptide has amino acids corresponding to amino acids 383 - 858 of the SEQ ID NO: 1.

In an embodiment, the variant polypeptide has amino acids corresponding to the amino acids 1 - 1165 of the SEQ ID NO: 1. In an embodiment, the variant polypeptide has amino acids corresponding to the amino acids 1 - 858 of the SEQ ID NO: 1. In an embodiment, the variant polypeptide has at least amino acids corresponding to the amino acids 383 - 858 of the SEQ ID NO: 1.

In an embodiment, the variant polypeptide has amino acids corresponding to the amino acids 1 - 1165 of the SEQ ID NO: 1 and has the size of 1165 amino acids. In an embodiment, the variant polypeptide has amino acids corresponding to the amino acids 1 - 858 of the SEQ ID NO: 1 and has the size of 858 amino acids. In an embodiment, the variant polypeptide has amino acids corresponding to the amino acids 383 - 858 of the SEQ ID NO: 1 and has the size of 476 amino acids. In an embodiment, the variant polypeptide has amino acids corresponding to the amino acids 1 - 858 of the SEQ ID NO: 1 and has the size of 858 - 1165 amino acids.

In an embodiment, the amino acid sequence of the variant polypeptide has at least 80 % amino acid sequence identity with amino acids 383 - 858 of SEQ ID NO: 1, and at least amino acids corresponding to the amino acids 383 - 858 or to amino acids 1 - 858 of the SEQ ID NO: 1. In a preferrable embodiment, the amino acid sequence of the variant polypeptide has at least 80 % amino acid sequence identity with amino acids 383 - 858 of SEQ ID NO: 1, and amino acids corresponding to the amino acids 1 - 1165 of the SEQ ID NO: 1.

In an embodiment, the variant polypeptide has amino acids corresponding to amino acids:

383 - 858, or 383 - 953, or 383 - 1118, or 383 - 1165, or
278 - 858, or 278 - 953, or 278 - 1118, or 278 - 1165, or
96 - 858, or 96 - 953, or 96 - 1118, or 96 - 1165, or
1 - 858, or 1 - 953, or 1 - 1118, or 1 - 1165;

of the SEQ ID NO: 1 and wherein the amino acids have at least 80 % amino acid sequence identity with the corresponding amino acids of the SEQ ID NO: 1.

In an embodiment, the variant polypeptide has amino acids corresponding to the amino acids 383 - 858 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 383 -953 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 383 - 1118 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 383 - 1165 of the SEQ ID NO: 1 wherein the amino acids have at least 80 % amino acid sequence identity with the corresponding amino acids of the SEQ ID NO: 1.

In an embodiment, the variant polypeptide has amino acids corresponding to the amino acids 278 - 858 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 278 -953 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 278 - 1118 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 278 - 1165 of the SEQ ID NO: 1 wherein the amino acids have at least 80 % amino acid sequence identity with the corresponding amino acids of the SEQ ID NO: 1.

In an embodiment, the variant polypeptide has amino acids corresponding to the amino acids 96 - 858 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 96 -953 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 96 - 1118 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 96 - 1165 of the SEQ ID NO: 1 wherein the amino acids have at least 80 % amino acid sequence identity with the corresponding amino acids of the SEQ ID NO: 1.

In an embodiment, the variant polypeptide has amino acids corresponding to the amino acids 1 - 858 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 1 -953 of the SEQ ID NO: 1 wherein the amino acids have at least 80 % amino acid sequence identity with the corresponding amino acids of the SEQ ID NO: 1. In an embodiment, the variant polypeptide has amino acids corresponding to the amino acids 1 - 1118 of the SEQ ID NO: 1 wherein the amino acids have at least 80 % amino acid sequence identity with the corresponding amino acids of the SEQ ID NO: 1. In a preferred embodiment, the variant polypeptide has amino acids corresponding to the amino acids 1 - 1165 of the SEQ ID NO: 1 wherein the amino acids have at least 80 % amino acid sequence identity with the corresponding amino acids of the SEQ ID NO: 1.

In an embodiment, the variant polypeptide consists of amino acids corresponding to the amino acids 1 - 858, or to the amino acids 1 - 953, or to the amino acids 1 - 1118, or to the amino acids 1 - 1165 of the SEQ ID NO: 1, wherein the amino acids have at least 80 % amino acid sequence identity with the corresponding amino acids of the SEQ ID NO: 1.

In an embodiment, the variant polypeptide has at least 85 %, or at least 90 %, or at least 95 %, or at least 98 %, amino acid sequence identity with the amino acids 383 - 858 of the SEQ ID NO: 1. In an embodiment, the variant polypeptide has at least 96 %, or at least 97 %, or at least 98 %, or at least 99 %, or at least 99.5 %, amino acid sequence identity with the amino acids 383 - 858 of the SEQ ID NO: 1.

In an embodiment, the variant polypeptide has at least 85 %, or at least 90 %, or at least 95 %, or at least 98 %, amino acid sequence identity with the amino acids 1 - 858 of the SEQ ID NO: 1. In an embodiment, the variant polypeptide has at least 96 %, or at least 97 %, or at least 98 %, or at least 99 %, or at least 99.5 % amino acid sequence identity with the amino acids 1 - 858 of the SEQ ID NO: 1.

In an embodiment, the variant polypeptide has at least 85 %, or at least 90 %, or at least 95 %, or at least 98 %, amino acid sequence identity with the amino acids 1 - 953 of the SEQ ID NO: 1. In an embodiment, the variant polypeptide has at least 96 %, or at least 97 %, or at least 98 %, or at least 99 %, or at least 99.5 % amino acid sequence identity with the amino acids 1 - 953 of the SEQ ID NO: 1.

In an embodiment, the variant polypeptide has at least 85 %, or at least 90 %, or at least 95 %, or at least 98 %, amino acid sequence identity with the amino acids 1 -1118 of the SEQ ID NO: 1. In an embodiment, the variant polypeptide has at least 96 %, or at least 97 %, or at least 98 %, or at least 99 %, or at least 99.5 % amino acid sequence identity with the amino acids 1 - 1118 of the SEQ ID NO: 1.

In an embodiment, the variant polypeptide has at least 85 %, preferably at least 90 %, more preferably at least 95 %, even more preferably at least 98 % amino acid sequence identity with the amino acids 1 - 1165 of the SEQ ID NO: 1. In an embodiment, the variant polypeptide has at least 96 %, or at least 97 %, or at least 98 %, or at least 99 %, or at least 99.5 %, amino acid sequence identity with the amino acids 1 - 1165 of the SEQ ID NO: 1.

In an embodiment, the variant polypeptide has amino acids corresponding to the amino acids 383 - 858 of the SEQ ID NO: 1 wherein the amino acids have at least 85 %, preferably at least 90 %, more preferably at least 95 %, even more preferably at least 98 % amino acid sequence identity with the amino acids 383 - 858 of the SEQ ID NO: 1. In an embodiment, the variant polypeptide has amino acids corresponding to the amino acids 1 - 858 of the SEQ ID NO: 1 wherein the amino acids have at least 85 %, preferably at least 90 %, more preferably at least 95 %, even more preferably at least 98 % amino acid sequence identity with the amino acids 1 - 858 of the SEQ ID NO: 1. In an embodiment, the variant polypeptide has amino acids corresponding to the amino acids 1 - 953 of the SEQ ID NO: 1 wherein the amino acids have at least 85 %, preferably at least 90 %, more preferably at least 95 %, even more preferably at least 98 % amino acid sequence identity with the amino acids 1 - 953 of the SEQ ID NO: 1. In an embodiment, the variant polypeptide has amino acids corresponding to the amino acids 1 - 1118 of the SEQ ID NO: 1 wherein the amino acids have at least 85 %, preferably at least 90 %, more preferably at least 95 %, even more preferably at least 98 % amino acid sequence identity with the amino acids 1 - 1118 of the SEQ ID NO: 1. In an embodiment, the variant polypeptide has amino acids corresponding to the amino acids 1 - 1165 of the SEQ ID NO: 1 wherein the amino acids have at least 85 %, preferably at least 90 %, more preferably at least 95 %, even more preferably at least 98 % amino acid sequence identity with the amino acids 1 - 1165 of the SEQ ID NO: 1. In an embodiment, the variant polypeptide has amino acids corresponding to the amino acids 96 - 1165 of the SEQ ID NO: 1 wherein the amino acids have at least 85 %, preferably at least 90 %, more preferably at least 95 %, even more preferably at least 98 % amino acid sequence identity with the amino acids 96 - 1165 of the SEQ ID NO: 1. In an embodiment, the variant polypeptide has amino acids corresponding to the amino acids 96 - 1118 of the SEQ ID NO: 1 wherein the amino acids have at least 85 %, preferably at least 90 %, more preferably at least 95 %, even more preferably at least 98 % amino acid sequence identity with the amino acids 96 - 1118 of the SEQ ID NO: 1. In an embodiment, the variant polypeptide has amino acids corresponding to the amino acids 96 - 953 of the SEQ ID NO: 1 wherein the amino acids have at least 85 %, preferably at least 90 %, more preferably at least 95 %, even more preferably at least 98 % amino acid sequence identity with the amino acids 96 - 953 of the SEQ ID NO: 1. In an embodiment, the variant polypeptide has amino acids corresponding to the amino acids 96 - 858 of the SEQ ID NO: 1 wherein the amino acids have at least 85 %, preferably at least 90 %, more preferably at least 95 %, even more preferably at least 98 % amino acid sequence identity with the amino acids 96 - 858 of the SEQ ID NO: 1.

In an embodiment, the variant polypeptide has amino acids corresponding to the amino acids 278 - 1165 of the SEQ ID NO: 1 wherein the amino acids have at least 85 %, preferably at least 90 %, more preferably at least 95 %, even more preferably at least 98 % amino acid sequence identity with the amino acids 278 - 1165 of the SEQ ID NO: 1. In an embodiment, the variant polypeptide has amino acids corresponding to the amino acids 278 - 1118 of the SEQ ID NO: 1 wherein the amino acids have at least 85 %, preferably at least 90 %, more preferably at least 95 %, even more preferably at least 98 % amino acid sequence identity with the amino acids 278 1118 of the SEQ ID NO: 1. In an embodiment, the variant polypeptide has amino acids corresponding to the amino acids 278 - 953 of the SEQ ID NO: 1 wherein the amino acids have at least 85 %, preferably at least 90 %, more preferably at least 95 %, even more preferably at least 98 % amino acid sequence identity with the amino acids 278 - 953 of the SEQ ID NO: 1. In an embodiment, the variant polypeptide has amino acids corresponding to the amino acids 278 - 858 of the SEQ ID NO: 1 wherein the amino acids have at least 85 %, preferably at least 90 %, more preferably at least 95 %, even more preferably at least 98 % amino acid sequence identity with the amino acids 278 - 858 of the SEQ ID NO: 1.

In an embodiment, the variant polypeptide has amino acids corresponding to the amino acids 383 - 1165 of the SEQ ID NO: 1 wherein the amino acids have at least 85 %, preferably at least 90 %, more preferably at least 95 %, even more preferably at least 98 % amino acid sequence identity with the amino acids 383 - 1165 of the SEQ ID NO: 1. In an embodiment, the variant polypeptide has amino acids corresponding to the amino acids 383 - 1118 of the SEQ ID NO: 1 wherein the amino acids have at least 85 %, preferably at least 90 %, more preferably at least 95 %, even more preferably at least 98 % amino acid sequence identity with the amino acids 383 1118 of the SEQ ID NO: 1. In an embodiment, the variant polypeptide has amino acids corresponding to the amino acids 383 - 953 of the SEQ ID NO: 1 wherein the amino acids have at least 85 %, preferably at least 90 %, more preferably at least 95 %, even more preferably at least 98 % amino acid sequence identity with the amino acids 383 - 953 of the SEQ ID NO: 1.

In an embodiment, the variant polypeptide has the amino acids 383 - 858 of the SEQ ID NO: 1. In an embodiment, the variant polypeptide has the amino acids 383 - 953 of the SEQ ID NO: 1. In an embodiment, the variant polypeptide has the amino acids 383 - 1118 of the SEQ ID NO: 1. In an embodiment, the variant polypeptide has the amino acids 383 - 1165 of the SEQ ID NO: 1.

In an embodiment, the variant polypeptide has the amino acids 278 - 858 of the SEQ ID NO: 1. In an embodiment, the variant polypeptide has the amino acids 278 - 953 of the SEQ ID NO: 1. In an embodiment, the variant polypeptide has the amino acids 278 - 1118 of the SEQ ID NO: 1. In an embodiment, the variant polypeptide has the amino acids 278 - 1165 of the SEQ ID NO: 1.

In an embodiment, the variant polypeptide has the amino acids 96 - 858 of the SEQ ID NO: 1. In an embodiment, the variant polypeptide has the amino acids 96 - 953 of the SEQ ID NO: 1. In an embodiment, the variant polypeptide has the amino acids 96 - 1118 of the SEQ ID NO: 1. In an embodiment, the variant polypeptide has the amino acids 96 - 1165 of the SEQ ID NO: 1.

In an embodiment, the variant polypeptide has the amino acids 1 - 858 of the SEQ ID NO: 1. In an embodiment, the variant polypeptide has the amino acids 1 - 953 of the SEQ ID NO: 1. In an embodiment, the variant polypeptide has the amino acids 1 - 1118 of the SEQ ID NO: 1. In an embodiment, the variant polypeptide has the amino acids 1 - 1165 of the SEQ ID NO: 1.

In an embodiment, the variant polypeptide has at least 100 or at least 150 amino acids deleted from the amino acids corresponding to the amino acids 859 - 1279 of the SEQ ID NO: 1, preferably having the at least 100 amino acids deleted from the amino acids corresponding to the amino acid 1166 - 1279 of the SEQ ID NO: 1.

In an embodiment, the variant polypeptide has at least 100 or at least 150 amino acids deleted from its C-terminal amino acid sequence or end. The variant polypeptide having said amino acids deleted from its C-terminal end is beneficial, as it has an increased fructanase productivity and/or improved fructan degrading performance, when compared to a polypeptide having the sequence SEQ ID NO: 1 or when compared to the wild type parent polypeptide of the variant polypeptide, preferably, the variant polypeptide has at least an increased fructanase productivity.

In an embodiment, the variant polypeptide has at least 100 or at least 150 amino acids deleted from the amino acids corresponding to the amino acids 859 - 1279 of the SEQ ID NO: 1. In an embodiment, the variant polypeptide has 100 - 421, or 150 - 421 amino acids deleted from the amino acids corresponding to the amino acids 859 - 1279 of the SEQ ID NO: 1. In an embodiment, the variant polypeptide has the amino acids corresponding to the amino acids 859 - 1279 of the SEQ ID NO: 1 deleted.

In an embodiment, the variant polypeptide has at least 100 or at least 150 amino acids deleted from the amino acids corresponding to the amino acids 954 - 1279 of the SEQ ID NO: 1. In an embodiment, the variant polypeptide has 100 - 326, or 150 - 326 amino acids deleted from the amino acids corresponding to the amino acids 954 - 1279 of the SEQ ID NO: 1. In an embodiment, the variant polypeptide has the amino acids corresponding to the amino acids 954 - 1279 of the SEQ ID NO: 1 deleted.

In an embodiment, the variant polypeptide has at least 100 or at least 150 amino acids deleted from the amino acids corresponding to the amino acids 1119 - 1279 of the SEQ ID NO: 1. In an embodiment, the variant polypeptide has 100 - 161, or 150 161 amino acids deleted from the amino acids corresponding to the amino acids 1119 - 1279 of the SEQ ID NO: 1. In an embodiment, the variant polypeptide has the amino acids corresponding to the amino acids 1119 - 1279 of the SEQ ID NO: 1 deleted.

In a preferable embodiment, the variant polypeptide has at least 100 amino acids deleted from the amino acids corresponding to the amino acid 1166 - 1279 of the SEQ ID NO: 1. In a preferable embodiment, the variant polypeptide has 100 - 114 amino acids deleted from the amino acids corresponding to the amino acids 1166 - 1279 of the SEQ ID NO: 1. In a preferable embodiment, the variant polypeptide has the amino acids corresponding to the amino acids 1166 - 1279 of the SEQ ID NO: 1 deleted.

In an embodiment, the variant polypeptide has at least 100 or at least 150 amino acids deleted from the amino acids corresponding to the amino acids 1 - 382 of the SEQ ID NO: 1. In an embodiment, the variant polypeptide has at least 100 or at least 150 amino acids deleted from its N-terminal amino acid sequence or end. In an embodiment, the variant polypeptide has, in addition to a C-terminal deletion of amino acids, also at least 100 or at least 150 amino acids deleted from its N-terminal amino acid sequence or end. In a preferable embodiment, the variant polypeptide has the at least 100 or at least 150 amino acids deleted from both C-terminal and N-terminal amino acid sequence or end of the variant polypeptide. The variant polypeptide having said amino acids deleted from its N-terminal end is beneficial, as it has an increased fructanase productivity and/or improved fructan degrading performance, when compared to a polypeptide having the sequence SEQ ID NO: 1 or when compared to the wild type parent polypeptide of the variant polypeptide, preferably, the variant polypeptide has at least an increased fructanase productivity.

In an embodiment, the variant polypeptide has at least 100 amino acids deleted from the amino acids corresponding to the amino acids 1 - 382 of the SEQ ID NO: 1. In an embodiment, the variant polypeptide has 100 - 382, or 150 - 382 amino acids deleted from the amino acids corresponding to the amino acids 1 - 382 of the SEQ ID NO: 1. In an embodiment, the variant polypeptide has the amino acids corresponding to the amino acids 1 - 382 of the SEQ ID NO: 1 deleted.

In an embodiment, the variant polypeptide has at least 100 amino acids deleted from the amino acids corresponding to the amino acids 1 - 277 of the SEQ ID NO: 1. In an embodiment, the variant polypeptide has 100 - 277, or 150 - 277 amino acids deleted from the amino acids corresponding to the amino acids 1 - 277 of the SEQ ID NO: 1. In an embodiment, the variant polypeptide has the amino acids corresponding to the amino acids 1 - 277 of the SEQ ID NO: 1 deleted.

In an embodiment, the variant polypeptide has the amino acids corresponding to the amino acids 1 - 95 of the SEQ ID NO: 1 deleted.

In an embodiment, the amino acid sequence of the variant polypeptide has at least 100 amino acids deleted in amino acid positions corresponding to the amino acid positions 1 - 382 and/or amino acid positions 858 - 1276 of SEQ ID NO: 1, wherein the at least 100 deleted amino acids is a sum of two or more deleted separate parts of the amino acid sequence. By separate parts of the amino acid sequence in this respect is meant stretches of the amino acid sequence, separated from each other by amino acids which are not deleted. This means, amino acids are deleted from two or more amino acid regions of the wild type parent polypeptide.

In an embodiment, the variant polypeptide is obtained via inactivation of at least one SLAP domain, or via deletion or substitution of amino acids of at least one SLAP domain of a fructanase enzyme having a sequence comprising at least one SLAP domain, the variant polypeptide thereby having at least one non-functional SLAP domain and/or at least one SLAP domain deleted.

As used herein, with the term "non-functional SLAP domain" is meant a SLAP domain that is not able to interact with other non-functional and/or functional SLAP domains, and which is optionally not able to anchor the fructanase enzyme to a cell wall.

In an embodiment, the variant polypeptide is obtained via inactivation of at least one SLAP domain of a fructanase enzyme having a sequence comprising at least one SLAP domain, the variant polypeptide thereby having at least one non-functional SLAP domain. In a preferable embodiment, the variant polypeptide is obtained via inactivation of two SLAP domains of a fructanase enzyme having a sequence comprising two SLAP domains, the variant polypeptide thereby having two non-functional SLAP domains. In an embodiment, the inactivation of at least one SLAP domain of a fructanase enzyme is obtained via a mutation introduced to the amino acid sequence of the fructanase enzyme, the mutation being selected form deletion and/or substitution and/or insertion of amino acids..

In an embodiment, the variant polypeptide is obtained via deletion or substitution of amino acids of at least one SLAP domain of a fructanase enzyme having a sequence comprising at least one SLAP domain, the variant polypeptide thereby having at least one non-functional SLAP domain and/or at least one SLAP domain deleted. In a preferable embodiment, the variant polypeptide is obtained via deletion or substitution of amino acids of two SLAP domains of a fructanase enzyme having a sequence comprising two SLAP domains, the variant polypeptide thereby having two non-functional SLAP domains and/or two SLAP domains deleted. In a further preferable embodiment, the variant polypeptide is obtained via deletion of amino acids of two SLAP domains of a fructanase enzyme having a sequence comprising two SLAP domains, the variant polypeptide thereby having two SLAP domains deleted.

In an embodiment, the size of the variant polypeptide is 1223 amino acids or less, and it is obtained via a C-terminal deletion of amino acids comprising one SLAP domain, the numbering of the deleted amino acids of the variant polypeptide corresponding to the amino acids 1224-1279 of the SEQ ID NO: 1. In an embodiment, the size of the variant polypeptide is 1228 amino acids or less, and it is obtained via a C-terminal deletion of amino acids comprising one SLAP domain, the numbering of the deleted amino acids of the variant polypeptide corresponding to the amino acids 1166 - 1216 of the SEQ ID NO: 1.

In an embodiment, the size of the variant polypeptide is 1165 amino acids or less, and it is obtained via a C-terminal deletion of amino acids comprising two SLAP domains, the numbering of the deleted amino acids of the variant polypeptide corresponding to the amino acid 1166-1279 of the SEQ ID NO: 1.

In an embodiment, the size of the variant polypeptide is 964 amino acids or less, and it is obtained via a C-terminal deletion of amino acids comprising Lam G and two SLAP domains, the numbering of the deleted amino acids of the variant polypeptide corresponding to the amino acids 965 - 1279 of the SEQ ID NO: 1.

In an embodiment, the size of the variant polypeptide is 867 amino acids or less, and it is obtained via a C-terminal deletion of amino acids comprising Big-2, Lam G and two SLAP domains, the numbering of the deleted amino acids of the variant polypeptide corresponding to the amino acids 868 - 1279 of the SEQ ID NO: 1.

In an embodiment, the size of the variant polypeptide is 858 amino acids or less, and it is obtained via a C-terminal deletion of amino acids comprising Big-2, Lam G and two SLAP domains, the numbering of the deleted amino acids of the variant polypeptide corresponding to the amino acids 859 - 1279 of the SEQ ID NO: 1.

In an embodiment, the size of the variant polypeptide is 590 amino acids or less, and it is obtained via a C-terminal deletion of amino acids comprising Big-2, Lam G and two SLAP domains; and an N-terminal deletion of amino acids comprising a Lam G domain, the numbering of the deleted amino acids of the variant polypeptide corresponding to the amino acids 868 - 1279 and 1-268 of the SEQ ID NO: 1.

In an embodiment, the size of the variant polypeptide is 482 amino acids or less, and it is obtained via a C-terminal deletion of amino acids comprising Big-2, Lam G and two SLAP domains; and an N-terminal deletion of amino acids comprising Lam G and Cadherine-like domains, the numbering of the deleted amino acids of the variant polypeptide corresponding to the amino acids 868 - 1279 and 1-376 of the SEQ ID NO: 1.

In an embodiment, the amino acid sequence of the variant polypeptide consists only of a GH32 domain. In an embodiment, the amino acid sequence of the variant polypeptide comprises a GH32 domain, and at least one domain selected from: one or two Lam G domains, a cadherine-like domain, a Big-2 domain, or any combination thereof (for reference, see a polypeptide of a full-length wild-type *Lactobacillus crispatus* LFRU fructanase in Fig. 1).

In an embodiment, the variant polypeptide having the size of at least 476 amino acids, but at most 1165 amino acids is beneficial as it has an increased fructanase productivity and/or increased fructan degrading performance, when compared to a polypeptide having the sequence SEQ ID NO: 1 or when compared to the wild type parent polypeptide of the variant polypeptide, preferably the variant has at least an increased fructanase productivity.

In an embodiment, the variant polypeptide is without one or more SLAP domains, or has at least one non-functional SLAP domain.

In an embodiment, the variant polypeptide has fructanase activity; an amino acid sequence having at least 80 % amino acid sequence identity with the amino acids 1-1165 of SEQ ID NO: 1, wherein the amino acid numbering of the amino acid sequence corresponds to the amino acid numbering of the SEQ ID NO: 1; and a size of 476 - 1165 amino acids, wherein the variant polypeptide does not contain one or more surface layer protein (SLAP) domains.

In an embodiment, the variant polypeptide has fructanase activity; an amino acid sequence having at least 80 % amino acid sequence identity with the amino acids 1-1165 of SEQ ID NO: 1, wherein the amino acid numbering of the amino acid sequence corresponds to the amino acid numbering of the SEQ ID NO: 1, and wherein the variant polypeptide does not contain an amino acid sequence as indicated in a PFAM family PF03217.

In an embodiment, the variant polypeptide is obtained via a deletion of SLAP domain amino acids of a fructanase enzyme having a sequence comprising at least one SLAP domain. In an embodiment, the variant polypeptide is obtained via a deletion of all SLAP domain amino acids of a fructanase enzyme having a sequence comprising one or more SLAP domains.

In an embodiment, the variant polypeptide is obtained via a C-terminal deletion of SLAP domain amino acids of a fructanase enzyme having a sequence comprising at least one C-terminal SLAP domain. In an embodiment, the variant polypeptide is obtained via an N-terminal deletion of SLAP domain amino acids of a fructanase enzyme having a sequence comprising at least one N-terminal SLAP domain. In a preferable embodiment, the variant polypeptide comprises no functional SLAP domains, more preferably, the variant polypeptide comprises no SLAP domains. In other words, the variant polypeptide is without functional SLAP domains, more preferably, the variant polypeptide is without SLAP domains.

In an embodiment, the SLAP domain amino acids of the variant polypeptide are deleted or inactivated from an amino acid region which does not overlap with the amino acids of the variant polypeptide corresponding to the amino acids 1-1165 of SEQ ID NO: 1.

In an embodiment, the variant polypeptide has fructanase activity; and a GH32 domain, two Lam G domains, a cadherine-like domain, and a Big-2 domain, the amino acids of said domains corresponding to the amino acids of SEQ ID NO: 1. In an embodiment, the variant polypeptide has fructanase activity; an amino acid sequence having the amino acids 1-1165 of SEQ ID NO: 1, wherein the amino acid numbering of the amino acid sequence corresponds to the amino acid numbering of the SEQ ID NO: 1.

In an embodiment, the variant polypeptide is of bacterial origin, meaning the wild type fructanase parent polypeptide of the variant polypeptide is a bacterial fructanase. In an embodiment, the variant polypeptide originates from a wild type bacterial fructanase of a family *Lactobacillaceae,* In an embodiment, the variant polypeptide originates from a wild type bacterial fructanase of a genus *Lactobacillus.* In an embodiment, the variant polypeptide originates from a wild type bacterial fructanase of the species *Lactobacillus crispatus Lactobacillus equi,* or *Lactobacillus amylovorus.*

In an embodiment, the variant polypeptide has an increased fructanase productivity and/or improved fructan degrading performance, when compared to a polypeptide having the sequence SEQ ID NO: 1.

For purposes of the present invention, fructanase productivity may be determined according to the method described in Example 2 below, and the fructan degrading performance (activity) may be determined according to the method described in Example 3 below.

In an embodiment, the variant polypeptide has an increased fructanase productivity and/or improved fructan degrading performance, when compared to a wild type parent polypeptide of the variant polypeptide. Preferably, the variant polypeptide has at least an increased fructanase productivity when compared to a polypeptide having the sequence SEQ ID NO: 1 or when compared to the wild type parent polypeptide of the variant polypeptide. In an embodiment, the variant polypeptide has an improved fructan degrading performance when compared to a polypeptide having the sequence SEQ ID NO: 1 or when compared to the wild type parent polypeptide of the variant polypeptide. In a preferred embodiment, with the variant polypeptide having at least one SLAP domain deleted or at least one non-functional SLAP domain is meant a variant polypeptide which has an increased fructanase productivity.

In an embodiment, the relative fructanase productivity of the variant polypeptide is at least 2, preferably at least 3, more preferably at least 4, more preferably at least 6, even more preferably at least 8 fold higher when compared to a wild type parent fructanase of the variant polypeptide, or to a polypeptide having the sequence according to SEQ ID NO: 1. In an embodiment, the relative fructanase productivity of the variant polypeptide is at least 10, preferably at least 50, more preferably at least 100 fold higher when compared to a wild type parent polypeptide of the variant polypeptide or to a polypeptide having the sequence according to SEQ ID NO: 1. In an embodiment, the wild type parent polypeptide of the variant polypeptide is not produced in any significant amounts, whereas the modifications comprised by the variant enable induced production of the variant polypeptide.

By fructanase productivity fold change, such as increase, in this respect is meant the ratio between the fructanase productivity of the measured variant (B) and fructanase productivity of the comparison sample (A), e.g. the polypeptide having the sequence SEQ ID NO: 1. Thus for fructanase productivities of (A) and (B) the fold change of B with respect to A is B/A.

In an embodiment, the increased fructanase productivity of the variant polypeptide means, the variant polypeptide also has an improved fructan degrading performance when compared to a polypeptide having the sequence SEQ ID NO: 1 or when compared to a wild type parent polypeptide of the variant polypeptide, as the absolute quantity of the variant polypeptide is increased.

In an embodiment, the variant polypeptide has an improved fructan degrading performance, when compared to a wild type parent fructanase of the variant polypeptide, or to a polypeptide having the sequence SEQ ID NO: 1, meaning, the variant polypeptide has a higher fructanase activity when compared to a wild type parent fructanase of the variant polypeptide, or to a polypeptide having the sequence SEQ ID NO: 1. In an embodiment, the variant polypeptide has an improved fructan degrading performance when compared to a wild type parent fructanase of the variant polypeptide, or to a polypeptide having the sequence SEQ ID NO: 1, wherein the improved fructan degrading performance of the variant polypeptide is independent of the host cell wherein the variant polypeptide is produced. In an embodiment, the variant polypeptide has an improved fructan degrading performance and/or improved fructanase activity when compared to a wild type parent fructanase of the variant polypeptide, or to a polypeptide having the sequence SEQ ID NO:1, at least when produced in a fungal or a bacterial cell.

In an embodiment, the relative fructanase activity of the variant polypeptide is at least 2, preferably at least 2,5 fold, more preferably at least 3 fold higher when compared to a wild type parent fructanase of the variant polypeptide, or to a polypeptide having the sequence SEQ ID NO: 1. By fructanase activity fold change, such as increase, in this respect is meant the ratio between the fructanase activity of the measured variant (B) and fructanase activity of the comparison sample (A), e.g. the polypeptide having the sequence SEQ ID NO: 1. Thus for fructanase activities of (A) and (B) the fold change of B with respect to A is B/A. In some embodiments, the variant polypeptide has an increased fructanase productivity but no significantly improved fructan degrading performance, when compared to a polypeptide having the sequence SEQ ID NO: 1, or when compared to a wild type parent polypeptide of the variant polypeptide.

In an embodiment, the variant polypeptide having the amino acid sequence having at least 80 % amino acid sequence identity with the amino acids 1-1165 of SEQ ID NO: 1 and a size of 476 - 1165 amino acids, has a higher fructanase productivity and/or activity, when compared to a wild type parent fructanase of the variant polypeptide, or to a polypeptide having the sequence SEQ ID NO: 1. In an embodiment, the variant polypeptide having an amino acid sequence having at least 80 % amino acid sequence identity with the amino acids 1-1165 of SEQ ID NO: 1 and a size of 476 - 1165 amino acids, has a higher fructanase productivity and/or activity, when compared to its wild type fructanase parent polypeptide having a size of more than 1165 amino acids.

In an embodiment, the variant polypeptide being obtained via a C-terminal deletion of amino acids 1166-1279 comprising two SLAP domains, has both, an increased fructanase productivity, and an increased fructanase activity, when compared to a wild type parent fructanase of the variant polypeptide, or to a fructanase polypeptide having the sequence SEQ ID NO: 1.

In an embodiment, the fructanase activity and/or productivity of the variant polypeptide is independent of the exact *Lactobacillus* species of origin of the variant polypeptide. In an embodiment, the relative fructanase activity and/or productivity of the variant polypeptide is independent of the exact *Lactobacillus* strain of origin of the variant polypeptide.

In an embodiment, the variant polypeptide is a variant of *Lactobacillus* fructanase. In an embodiment, the variant polypeptide is a variant of *Lactobacillus crispatus* fructanase or *Lactobacillus amylovorus* fructanase. In an embodiment, fructan degrading performance and/or fructanase productivity of the variant polypeptide is higher when compared to its parent fructanase polypeptide comprising amino acids corresponding to the amino acids of sequence SEQ ID NO: 1.

In an embodiment, the variant polypeptide has a substrate specificity to levan over inulin. In an embodiment, the variant polypeptide has a substrate specificity ratio of inulin:levan of 0.8 or less, preferably 0.5 or less, more preferably 0.4 or less or 0.3 or less. In an embodiment, the variant polypeptide has a pH optimum of 4 - 7, preferably of 5 - 6. In an embodiment, the variant polypeptide has a temperature optimum of 40 - 70 °C, preferably of 50 - 70 °C, such as 60 °C. In an embodiment, the variant polypeptide has a substrate specificity ratio of inulin:levan of 0.8 or less, preferably 0.5 or less; and/or a pH optimum of 5-6; and/or a temperature optimum of 50 - 70 °C. The above-mentioned properties show that the variant polypeptide is stable and active over a wide temperature and pH range. In an embodiment, the variant polypeptide which has fructanase activity and which retains activity in temperatures above ambient temperature is advantageous for applications wherein fructan degradation is required in such process conditions. High temperature tolerance of fructanase has the benefit of increased specific activity.

In an embodiment the variant polypeptide has a predicted molecular weight between 80 - 150 kDa, preferably between 100 - 150 kDa, comprising the amino acids constituting the mature region of the variant polypeptide and without including a signal sequence. In an embodiment the variant polypeptide has a predicted molecular weight of approximately 130 - 145 kDa, wherein the variant is without the SLAP domains of its parent polypeptide. In an embodiment the variant polypeptide has a predicted molecular weight of approximately 80 - 90 kDa, wherein the variant has the C-terminal SLAP, Lam G, and Big2 domains of its parent polypeptide removed. The predicted molecular weight can be determined by calculating the sum of the molecular weights of the individual amino acids in the variant polypeptide.

The present variant of fructanase is advantageous in having good stability and fructanase productivity. The variant has a different size and secretion pattern compared to a wild type fructanase, which may provide improved productivity and yield when produced in a host cell. Thus, the present variant of fructanase may provide improved yield in production and better performance in use. Thus, the present variant of fructanase is advantageous in having improved productivity. The present variant of fructanase may be advantageous also in having an increased activity. As shown in the examples, the claimed variant polypeptide of fructanase has an activity and level of productivity, which are improved when compared to the wild type parent fructanase.

In an embodiment, a fusion protein comprises the variant polypeptide, and at least one: an amino acid sequence providing a secretory signal sequence; an amino acid sequence which facilitates purification, such as an affinity tag or His-tag; an amino acid sequence controlling and enhancing the production of the fusion protein, such as a carrier polypeptide; a linker sequence; an amino acid sequence encoding a protease cleavage site; an amino acid sequence having an enzyme activity; and/or an amino acid sequence providing for the fusion protein with binding affinity, such as a carbohydrate binding moiety.

In an embodiment, the fusion protein comprises the amino acid sequence of the variant polypeptide and the at least one amino acid sequence providing a secretory signal sequence, said secretory signal sequence directing the fusion protein to secretory pathway of the host cell wherein the fusion protein is produced. In an embodiment, the fusion protein comprises a signal sequence which is advantageous in the translocation of the variant polypeptide within and out of the host cell. In an embodiment, the signal sequence is with or without a carrier polypeptide sequence. In an embodiment, the secretory signals is recombinant host cell specific.

In an embodiment, the fusion protein comprises an amino acid sequence which facilitates purification, such as His-tag or polyhistidine tag, wherein in the tag comprises a string of histidine residues. In an embodiment, the fusion protein comprises one or more sequences that control and direct the production of the variant polypeptide of fructanase.

In an embodiment, the fusion protein comprises a carbohydrate binding moiety (CBM) as a carrier polypeptide, which is optionally a fragment of another protein or enzyme derived from the same or different organism as the variant polypeptide. The signal sequence and carrier polypeptide may be derived from the same host, or alternatively derived from different hosts. In an embodiment, the fusion protein comprises a signal sequence originating from the same host cell wherein the fusion protein comprising the fructanase variant polypeptide is produced in. In an embodiment the signal sequence originates from *Bacillus subtilis,* wherein the fusion protein comprising the fructanase variant polypeptide is produced in. In another embodiment, the signal sequence originates from another organism than the host cell wherein the fusion protein comprising the fructanase variant polypeptide is produced in.

In an embodiment, the fusion protein comprises an amino acid sequence encoding a protease cleavage site. In an embodiment, the fusion protein comprises an amino acid linker sequence, linking two domains or parts of the fusion protein together.

In an embodiment, the fusion protein comprises an amino acid sequence having an enzyme activity, the said sequence with enzyme activity not being the same sequence as the amino acid sequence encoding the variant polypeptide with fructanase activity.

In an embodiment, the fusion protein comprises a carbohydrate binding moiety amino acid sequence, which provides the fusion protein with binding affinity. In an embodiment, the fusion protein comprises a stabilizer.

In an embodiment, an enzyme composition comprises the variant polypeptide or the fusion protein. In an embodiment, an enzyme composition comprises at least the present variant polypeptide, and optionally a preservative, and/or a carrier. In an embodiment the enzyme composition comprises the variant polypeptide or the fusion protein, and optionally one or more other polypeptides with enzymatic activity. In an embodiment, in addition to the variant polypeptide or the fusion protein, the enzyme composition may also comprise one or more enzymes having hydrolase activity.

In an embodiment the enzyme composition comprising the variant polypeptide of fructanase or the fusion protein, and one or more additional enzymes is advantageous in providing a synergistic effect. Such an additional enzyme is desired when the present enzyme composition comprising variant polypeptide or the fusion protein is used in e.g., preparation of food products. Therefore, in an embodiment, the present fructanase enzyme is blended with other enzymes thereby obtaining the enzyme composition, to be used in baking, food, feed, and/or technical applications, for example.

Particularly advantageous synergistic enzymes that work with fructanase in food products are inulinases, levanases, endo-fructanases and invertases. Further particularly advantageous synergistic enzymes that work with fructanase in food applications such as baking applications are glucoamylases. In an embodiment, the enzyme composition comprising the variant polypeptide of fructanase or the fusion protein comprises an additional enzyme such as an enzyme liberating glucose or maltose. In an embodiment, the enzyme composition comprising the variant polypeptide of fructanase or the fusion protein comprises one or more enzymes selected from the group consisting of amylase, maltogenic amylase, beta amylase, aminopeptidase, carboxypeptidase, catalase, cellulolytic enzyme, chitinase, cutinase, cyclodextrin glycosyltransferase, deoxyribonuclease, esterase, glucan 1,4- alpha-maltotetrahydrolase, glucanase, galactanase, alpha-galactosidase, beta-galactosidase, glucoamylase, glucose oxidase, alpha-glucosidase, beta-glucosidase, haloperoxidase, hemicellulolytic enzyme, invertase, laccase, lipase, mannanase, mannosidase, oxidase, pectinolytic enzymes, peptidoglutaminase, peroxidase, phospholipase, phytase, polyphenoloxidase, proteolytic enzyme, ribonuclease, transglutaminase, xylanase, glucose isomerase, mannitol dehydrogenase and fructose dehydrogenase.

In an embodiment, the enzyme composition comprising the variant polypeptide of fructanase or the fusion protein comprises at least one additional enzyme, such as an enzyme used in baking, animal feed, hygiene product or in food, one or more other fructan degrading enzymes, sugar isomerases, sugar transferases and/or sugar dehydrogenases.

In an embodiment, the enzyme composition is a liquid composition comprising diluents such as water or buffer, and/or stabilizers, such as sorbitol, glycerol or propylene glycol, and/or preservatives, such as sodium benzoate, potassium sorbate or parabens.

In an embodiment, the enzyme composition is provided in the form of a liquid composition or a solid composition, or mixtures thereof. In an embodiment, the enzyme composition is provided in the form of a solution, dispersion, paste, flour, powder, granule, granulate, coated granulate, tablet, cake, crystal, crystal slurry, gel, or pellet.

The present variant polypeptide, the present fusion protein and/or the present enzyme composition is advantageous, as it has increased activity and/or productivity, when compared, for example, to a polypeptide according to SEQ ID NO: 1. The present variant polypeptide, the present fusion protein and the present enzyme composition are advantageous, for example, for the degradation of fructan in grain materials, cereals, vegetables, and fruits.

The present variant polypeptide is preferably recombinantly produced non-naturally occurring protein. It can be prepared using generally known recombinant DNA technology. Briefly, the polynucleotide encoding a variant polypeptide is cloned and inserted into an expression vector, transformed into a host cell, and then expressed. Preferably, mutations are introduced into the coding sequence with codons preferred by the selected host strain. Methods for protein production by recombinant technology in different host systems are known in the art. Preferably, the variants are produced as extracellular proteins that are secreted into the culture medium wherein the host cell is cultured, and from which they can be easily recovered and isolated.

In an embodiment, a recombinant host cell comprises genetic elements that allow producing at least the variant polypeptide or the fusion protein. In an embodiment, the recombinant host cell is a microorganism, selected from the group consisting of eukaryotic fungal cells or prokaryotic bacterial cells, preferably the host cell is selected from filamentous fungal cells such as *Trichoderma* or *Aspergillus,* or from gram-positive Bacilli such as *Bacillus,* most preferably the host cell is *Bacillus.*

In an embodiment, the recombinant host cell is a fungal cell, preferably a filamentous fungal cell such as *Trichoderma, Aspergillus , Thermothelomyces, Myceliophthora , Humicola,* or *Fusarium.* In an embodiment, the recombinant host cell is a filamentous fungal cell such as *Trichoderma reesei, Aspergillus oryzae, Aspergillus niger, Thermothelomyces heterothallica, Myceliophthora thermophila, Humicola insolens,* or *Fusarium venenatum.*

In an embodiment, the recombinant host cell is a gram-positive Bacilli such as *Bacillus,* in particular *Bacillus subtilis, Bacillus pumilus, Bacillus alkalophilus, Bacillus amyloliquefaciens, Bacillus brevis, Bacillus circulans, Bacillus clausii, Bacillus coagulans, Bacillus halodurans, Bacillus lautus, Bacillus lentus, Bacillus licheniformis, Bacillus megaterium, Bacillus stearothermophilus,* or *Bacillus thuringiensis.*

In an embodiment, the recombinant host cell is a gram-negative bacterium such as *Escherichia coli* and/or an actinomycetale such as *Streptomyces sp.,* or *Nonomuraea flexuosa.* In an embodiment, the recombinant host cell is a yeast cell such as *Saccharomyces cerevisiae, Schizosaccharomyces pombe, Pichia pastoris, Yarrowia lipolytica.*

In a preferable embodiment, the host cell belongs to the genus *Bacillus.* In a preferable embodiment, the host cell is *Trichoderma reesei* or *Bacillus subtilis,* more preferably the host cell is *Bacillus subtilis*.

In an embodiment, the recombinant host cell has an increased fructanase productivity when compared to a host cell comprising genetic elements that allow producing the wild type parent polypeptide of the variant polypeptide, or when compared to a host cell comprising genetic elements that allow producing a polypeptide having the sequence SEQ ID NO: 1.

In an embodiment, the recombinant host cell comprises genetic elements that allow producing the at least one variant polypeptide or the fusion protein comprising the amino acid sequence of the variant polypeptide, wherein the recombinant host cell has an increased fructanase secretion when compared to a host cell comprising genetic elements that allow producing a polypeptide or a fusion protein comprising the sequence SEQ ID NO: 1, or when compared to a host cell comprising genetic elements that allow producing the wild type parent polypeptide of the variant polypeptide.

In an embodiment, the relative fructanase productivity of the recombinant host cell is at least 2 fold higher when compared to a host cell comprising genetic elements that allow producing a polypeptide according to SEQ ID NO: 1, or when compared to a host cell comprising genetic elements that allow producing the wild type parent polypeptide of the variant polypeptide. In an embodiment, the relative fructanase productivity of the variant polypeptide is at least 3, preferably at least 4, more preferably at least 6, even more preferably at least 8 fold higher, when compared to a to a host cell comprising genetic elements that allow producing a polypeptide according to SEQ ID NO: 1, or when compared to a host cell comprising genetic elements that allow producing the wild type parent polypeptide of the variant polypeptide. In a preferable embodiment, the relative fructanase productivity of the variant polypeptide is at least 10, preferably at least 50, more preferably at least 100 fold higher, when compared to a to a host cell comprising genetic elements that allow producing a polypeptide according to SEQ ID NO: 1, or when compared to a host cell comprising genetic elements that allow producing the wild type parent polypeptide of the variant polypeptide. In an embodiment, the relative fructanase productivity of the recombinant host cell refers to the quantity of the fructanase variant polypeptide produced into the production medium.

The recombinant host cell can be used to produce the variant polypeptide of fructanase and to contain a polynucleotide encoding it. The selection of a specific recombinant host cell can be made in preparation of variants of fructanase with different properties. For example, a host cell can be selected, which provides post-translational modifications beneficial for stability or activity, or which facilitates processing of the variant polypeptide of fructanase produced in the host cell. The host cell can be operably linked to one or more control sequences to direct the production of the variant, and that make it possible to initiate the production of the present variant polypeptide by a stimulus, as is known in the field. In an embodiment the host cell is non-pathogenic. This is particularly advantageous when using the host cell, or the fructanase variant produced in it, for foodstuff, or feedstuff applications.

The present recombinant host cell is advantageous, as it has an increased productivity of the present variant polypeptide and/or the present fusion protein, when compared to, for example, a host cell comprising the polypeptide according to SEQ ID NO: 1. The present recombinant host cell is advantageous, as it produces the present variant polypeptide and/or the present fusion protein having fructanase activity.

In an embodiment, a method of manufacturing the variant polypeptide or the fusion protein, comprises: cultivating the recombinant host cell in conditions allowing production of the at least one variant polypeptide, or the fusion protein; and optionally recovering the variant polypeptide or the fusion protein.

In an embodiment of the method, the recombinant host cells are separated from the medium comprising the variant polypeptide and/or the fusion protein, an optionally, said medium is concentrated by removing water. The concentrate can be made, for example, by ultrafiltration, microfiltration, evaporation or any other suitable means. In an embodiment, the variant polypeptide and/or the fusion protein is separated from other proteins, optionally this is done by selective crystallization, precipitation, chromatographic methods, or any other suitable means.

The present method of manufacturing the variant polypeptide or the fusion protein is advantageous, as it allows production of the variant polypeptide or the fusion protein having increased fructanase productivity and/or improved fructan degrading performance, when compared to, for example, the wild type parent polypeptide of the variant polypeptide or a polypeptide having the sequence SEQ ID NO: 1.

In an embodiment, the variant polypeptide, and/or the fusion protein, and/or the enzyme composition is used for degradation of fructan in plant-based material. In an embodiment, the use of the variant polypeptide and/or the fusion protein and/or the enzyme composition refers to method of using.

In an embodiment, the use comprises bringing the variant polypeptide and/or the fusion protein and/or the enzyme composition in contact with fructan comprised by the plant-based material, thereby allowing the degradation of fructan comprised by said plant-based material into at least fructose. In an embodiment, the use comprises bringing the variant polypeptide and/or the fusion protein and/or the enzyme composition in contact with fructan comprised by the plant-based material, at reaction conditions allowing the degradation of fructan by the variant polypeptide. In an embodiment, plant-based material is fructan containing material. In an embodiment, plant-based material is fructan containing material. In an embodiment, the fructan containing materials comprise:

cereals, such as wheat, spelt, rye and barley;

vegetables such as onions, shallots, garlic, leeks, artichoke, asparagus, beets, brussels sprouts, savoy cabbage, fennel, chicory, agave and snow peas;

nuts, such as cashews and pistachios;

pulses, such as kidney beans, black beans, mung means, navy beans, lima beans and split peas; and

fruits such as watermelon, nectarine, grapefruit, persimmon, pomegranate, plums, ripe bananas, dates, prunes and raisins.

In an embodiment, the variant polypeptide and/or the fusion protein and/or the enzyme composition can be used for the degradation of fructan in fructan containing materials. In an embodiment, the variant polypeptide and/or the fusion protein and/or the enzyme composition can be used for the degradation of fructan in grain and cereal containing materials, such as baked products including breads and cakes. In an embodiment, the variant polypeptide and/or the fusion protein and/or the enzyme composition can be used for the degradation of fructan in vegetable and/or fruit containing foods, such as vegetable containing foodstuff preparations.

In an embodiment, the fructan containing materials comprise processed food and/or convenience food.

In an embodiment, the use of the variant polypeptide and/or the fusion protein and/or the enzyme composition reduces fructan content of the fructan containing material at least 20 %, preferably at least 30 %, more preferably at least 40 %, even more preferably at least 50 %, most preferably at least 60 %, when compared to the fructan containing material without any fructanase enzyme.

In an embodiment, the variant polypeptide and/or the fusion protein and/or the enzyme composition is used to liberate fructose from fructan. In an embodiment, the use of the variant polypeptide and/or the fusion protein and/or the enzyme composition increases the fructose and glucose content of the fructan containing material, when compared to the fructan containing material without any fructanase enzyme. In an embodiment, the variant polypeptide and/or the fusion protein and/or the enzyme composition reduces the fructan content and increases the fructose content of the fructan containing material when compared to the fructan containing material without any fructanase enzyme.

In an embodiment, the use of the variant polypeptide and/or the fusion protein and/or the enzyme composition comprises a use of a combination of the present variant fructanase and a glucoamylase for degradation of fructan in plant-based material, for keeping the ratio of fructose / glucose constant in the enzyme composition treated plant-based material. In an embodiment, the variant polypeptide and/or the fusion protein and/or the enzyme composition, is brought in contact with plant-based material together with glucoamylase, thereby degrading fructan and keeping the ratio of fructose / glucose constant in the plant-based material. In such an embodiment, the use, or the method wherein the variant polypeptide and a glucoamylase are used in combination, is a use in baking application, for example.

In an embodiment, the variant polypeptide and/or the fusion protein and/or the enzyme composition is used to degrade fructans in plant-based material, to obtain foodstuff suitable for a diet low in fermentable carbohydrates, known as FODMAP. In an embodiment, the use of the variant polypeptide and/or the fusion protein and/or the enzyme composition is especially beneficial in preparing products comprising plant-based materials suitable for diet low in FODMAP ("Fermentable, Oligo-, Di-, Monosaccharides, and Polyols"), the materials thereby having low fructan content.

In an embodiment, the variant polypeptide and/or the fusion protein and/or the enzyme composition is used to degrade fructans in plant-based material, to obtain fructose sirup, which fructose sirup can be used for further applications, such as alcohol fermentation. In such an embodiment, the use comprises adding the present variant polypeptide and/or the fusion protein and/or the enzyme composition to the application in question, and eventually recovering fructose sirup.

In an embodiment, the use or method of using comprises the use of the variant polypeptide and/or the fusion protein and/or the enzyme composition in baking, manufacturing feedstuff, foodstuff, feed additive, a dietary supplement, a pharmaceutical, a detergent, a cleaning product, a hygiene product (such as mouth wash) processed food, animal feed (such as horse feed). In such embodiment, the use comprises adding the present variant polypeptide and/or the fusion protein and/or the enzyme composition to the application in question, such as baking, and mixing.

In an embodiment, the variant polypeptide and/or the fusion protein and/or the enzyme composition is added to the plant-based material, such as flour or dough, in an amount of 0.5-500 mg (corresponding approximately to 10 - 10000 fructanase units, FRU) of the variant polypeptide per kg of the plant-based material. In an exemplary embodiment, the variant polypeptide is added in an amount of 5-100 mg of the variant polypeptide per kg of flour, more preferably in an amount of 20-70 mg of the variant polypeptide per kg of flour.

In an embodiment, the variant polypeptide and/or the fusion protein and/or the enzyme composition is used in preparation artificial sweeteners, such as low-calorie sweeteners (LCS). In an embodiment, the variant polypeptide and/or the fusion protein and/or the enzyme composition is used in preparation of fructose, which is beneficial at least because of low production costs. In an embodiment, the variant polypeptide and/or the fusion protein and/or the enzyme composition is used in manipulating fructan metabolism in plants.

Using the present variant polypeptide and/or the present fusion protein and/or the present enzyme composition is advantageous, as the variant polypeptide is capable of increasing degradation of fructan.

In an embodiment, there is provided a premix for baking, comprising the variant polypeptide, and/or the fusion protein, and/or the enzyme composition. In an embodiment, the premix for baking comprises at least the variant polypeptide and/or the fusion protein and/or the enzyme composition, and one or more ingredients needed or suitable for baking. In an embodiment, the ingredients needed or suitable for baking include ingredients selected from one or more of flour, cereal grains or flakes, sugar, yeast or other leavening agents, fat or oil, salt, spices, flavorings, bakery ingredients, water, milk or milk alternative and eggs . In an embodiment, the ingredients needed or suitable for baking also include an improver for baking, comprising one or more ingredients from the group consisting of enzymes, wheat gluten, carriers (wheat gluten maltodextrin etc.), emulsifiers, and mono and diglycerides.

The present variant polypeptide, the present fusion protein and the present enzyme composition are advantageous in the premix for baking as the variant polypeptide is capable of increasing degradation of fructan in the premix and/or in the baking dough wherein the premix is mixed into. This is especially beneficial in preparing baked products suitable for LOW-FODMAP diet, having low fructan content.

In an embodiment, the variant polypeptide has fructanase activity; an amino acid sequence having at least 80 % amino acid sequence identity with the amino acids 1-1165 of SEQ ID NO: 1; and at least one SLAP domain deleted or at least one non-functional SLAP domain, obtained via deletion or substitution of amino acids, wherein the amino acid numbering of the variant polypeptide corresponds to the amino acid numbering of the SEQ ID NO: 1. In an embodiment, the variant polypeptide has at least one SLAP domain deleted via deletion or substitution of amino acids, preferably the variant polypeptide has two SLAP domains deleted. In an embodiment, the variant polypeptide has at least one partial and non-functional SLAP domain, the functionality being destroyed via at least one deletion or substitution of amino acids of the at least one SLAP domain. In an embodiment, the variant polypeptide has two partial and non-functional SLAP domains. In an embodiment, the variant polypeptide has one partial and non-functional SLAP domain and one SLAP domain deleted via deletion or substitution of amino acids. In an embodiment, the variant polypeptide is without at least one SLAP domain, the variant polypeptide thereby having at least one SLAP domain deleted, preferably two SLAP domains deleted.

In an embodiment, the variant polypeptide has at least one non-functional SLAP domain obtained via deletion of amino acids within the SLAP domain amino acid sequence. In an embodiment, the variant polypeptide has at least one non-functional SLAP domain obtained via substitution of amino acids of the SLAP domain amino acid sequence. In an embodiment, the variant polypeptide has at least one non-functional SLAP domain obtained via deletion and/or substitution of amino acids of the SLAP domain amino acid sequence.

In a preferred embodiment, the variant polypeptide has fructanase activity; an amino acid sequence having at least 80 % amino acid sequence identity with the amino acids 1-1165 of SEQ ID NO: 1; and two SLAP domains deleted or two non-functional SLAP domains, obtained via deletion or substitution of amino acids, wherein the amino acid numbering of the variant polypeptide corresponds to the amino acid numbering of the SEQ ID NO: 1.

In an embodiment, the variant polypeptide has at least 85 %, preferably at least 90 %, more preferably at least 95 %, even more preferably at least 98 % amino acid sequence identity with the amino acids 1 - 1165 of the SEQ ID NO: 1. In an embodiment, the variant polypeptide has an amino acid sequence comprising amino acids 1 - 1165 having at least 84 %, at least 86 %, at least 88 %, at least 90 %, at least 92 %, at least 94 %, at least 96 %, at least 98 % or even 100 % amino acid sequence identity with the amino acids 1-1165 of SEQ ID NO: 1, wherein the amino acid numbering of the amino acid sequence corresponds to the amino acid numbering of the SEQ ID NO: 1.

In an embodiment, the amino acid sequence of the variant polypeptide has less than 20 %, preferably less than 10 %, more preferably less than 5 % amino acid sequence identity with the amino acids 1166 - 1276 of SEQ ID NO: 1; and at least one SLAP domain deleted or at least one non-functional SLAP domain, obtained via deletion or substitution of amino acids, wherein the amino acid numbering of the variant polypeptide corresponds to the amino acid numbering of the SEQ ID NO: 1. In an embodiment, the variant polypeptide does not have amino acids corresponding to the amino acids 1166 - 1216; or to the amino acids 1224 - 1276; or to the amino acids 1166 - 1276 of SEQ ID NO: 1.

In an embodiment, the variant polypeptide of eight aspect, having the at least one SLAP domain deleted and/or at least one non-functional SLAP domain, and the variant polypeptide of the first aspect having the size of 476 - 1165 amino acids, are corresponding features of said variants. In an embodiment, the variant polypeptide of eight aspect, having the at least one SLAP domain deleted and/or at least one non-functional SLAP domain, achieves the same technical effect as the variant polypeptide of the first aspect having the size of 476 - 1165 amino acids, wherein the technical effect is at least increased fructanase productivity and/or improved fructan degrading performance. Thus, the variant polypeptide of eight aspect and the variant polypeptide of the first aspect are linked, thereby forming a single general inventive concept.

In an embodiment, there is provided a method of reducing fructan content in a baked product, the method comprising:

- providing ingredients for baking and mixing said ingredients together with the variant polypeptide and/or the fusion protein and/or the enzyme composition, thereby providing a dough, followed by proofing the dough, the fructan content in the dough being reduced while mixing and proofing; and
- baking the dough thereby providing the baked product having reduced fructan content.

In an embodiment, the method of reducing fructan content in a baked product comprises mixing the variant polypeptide and/or the fusion protein and/or the enzyme to the baking ingredients in an amount of 0.5-500 mg (corresponding approximately to 10 - 10000 fructanase units, FRU) of the variant polypeptide per kg of the plant-based material, such as flour, comprised by the baking ingredients. In an exemplary embodiment, the variant polypeptide is added in an amount of 5-100 mg of the variant polypeptide per kg of flour, more preferably in an amount of 20-70 mg of the variant polypeptide per kg of flour.

The dough produced with said method also has a reduced fructan content, the method thus providing also a method for reducing fructan content in a non-baked product such as dough.

In an embodiment, there is provided a food or foodstuff, comprising the variant polypeptide and/or the fusion protein and/or the enzyme composition. In an embodiment, the food or foodstuff, comprising the variant polypeptide and/or the fusion protein and/or the enzyme composition, is a mixture food product and/or processed food product, such as manufactured plant-based product/processed plant-based product, a supplement, an additive or mixtures thereof.

In an embodiment, there is provided a baked product having reduced fructan content, the baked product comprising the variant polypeptide and/or the fusion protein and/or the enzyme composition, and wherein the fructan content is reduced compared to a baked product without said variant polypeptide and/or the fusion protein and/or the enzyme composition. In an embodiment, fructan content of the baked product is reduced at least 20 %, at least 30 %, at least 40 %, or at least 50 %, when compared to a baked product which does not comprise said variant polypeptide and/or the fusion protein and/or the enzyme composition.

In an embodiment, the baked product comprises the variant polypeptide and/or the fusion protein and/or the enzyme to the baking ingredients in an amount of 0.5-500 mg of the variant polypeptide per kg of the plant-based material, such as flour, comprised by the baked product. In an exemplary embodiment, the baked product comprises 5-100 mg of the variant polypeptide per kg of flour, more preferably in an amount of 20-70 mg of the variant polypeptide per kg of flour.

In an embodiment, there is provided an animal feed or feedstuff, comprising the variant polypeptide and/or the fusion protein and/or the enzyme composition. In an embodiment, the animal feed or feedstuff, comprising the variant polypeptide and/or the fusion protein and/or the enzyme composition, is a dry forage, roughage, pasture, silage, energy feed, protein supplement, mineral supplement, vitamin supplement, additive, or mixtures thereof. In an embodiment the feed is animal feed intended to be fed to animals, such as any compound feed or mixture. In another embodiment feed comprises grains such as maize, wheat, oats, barley, sorghum and rice; protein sources such as soybean meal, sunflower meal and canola meal; and/or one or more minerals.

In an embodiment, there is provided a detergent comprising the variant polypeptide and/or the fusion protein and/or the enzyme composition. In an embodiment, the detergent is provided in the form of a solution, dispersion, paste, powder, granule, granulate, coated granulate, tablet, cake, crystal, crystal slurry, gel, or pellet.

## EXAMPLES

The following examples are provided to better illustrate the claimed invention. The examples are not to be interpreted as limiting the scope of the invention, which is determined by the appended claims. To the extent that specific materials are mentioned, it is merely for purposes of illustration and not intended to limit the invention. One skilled in the art may develop equivalent means or reactants without exercising inventive capacity and without departing from the scope of the invention. It shall be understood that many variations can be made in the procedures described herein while remaining within the scope of the present invention.

### Example 1. Fructanase variant design

A variant polypeptide was designed based on the wildtype *Lactobacillus crispatus* LFRU fructanase disclosed in WO2017220864. The variant is designed to have increased fructanase activity and/or productivity, and to be produced, for example, in *Bacillus subtilis*. The resulting variant polypeptide comprises multiple putative domains in addition to the GH32 catalytic domain (the domain structure of the wild type parent fructanase presented in Figure 1). The variant polypeptide was designed based on the wildtype *Lactobacillus crispatus* LFRU fructanase consisting of 1279 amino acids (SEQ ID NO:1). The two C-terminal SLAP domains were removed from the variant by deleting the terminal 114 amino acids, the variant polypeptide thereby ending at serine in position 1165, resulting in variant polypeptide having the SEQ ID NO: 2 (numbering starting from the amino acid sequence presented in SEQ ID NO: 1).

### Example 2. Production of fructanase variants in *Bacillus subtilis*

Standard molecular biology methods were used in the isolation and enzyme treatments of DNA (e.g., isolation of plasmid DNA, digestion of DNA to produce DNA fragments), sequencing etc. The basic laboratory methods used were either as described by the enzyme, reagent or kit manufacturer or as described in the standard molecular biology handbooks, e.g., Sambrook and Russell (2001) or as described in the following examples.

Both, a nucleotide sequence encoding for the wildtype LFRU fructanase polypeptide (SEQ ID NO:1) and a nucleotide sequence encoding for the truncated variant without S-layer domains (SEQ ID NO: 2), were cloned into a pUB110 derivate with an amylase promoter from *Bacillus amyloliquefaciens* by PCR using Phusion Polymerase (Thermo Scientific). The following PCR conditions were used: 120 sec initial denaturation at 94 °C, followed by 35 cycles of 15 sec at 94 °C, 30 sec annealing at 48 °C, 110/290 sec extension at 68 °C and the final extension at 68 °C for 10 min. For amplification of a LFRU fructanase, a synthetic gene was ordered without codon optimization (Genewiz, Germany). Oligonucleotides used for cloning are shown in Table 1.

**Table 1.** Oligonucleotides for generation of expression vectors. Overhangs for hybridization are underlined.

| Description | 5' à 3' | SEQ ID |
|---|---|---|
| Vector_fw | GTTTCCTCTCCCTCTCATTTTC | 3 |
| Vector_rev | TAAAGAGATATACCGACAGTG | 4 |
| Fru_fw1 | ATGAGAGGGAGAGGAAACATGAGTCATAAATTTAATAATTACGG | 5 |
| Fru_rev1 | GGTATATCTCTTTAAGACTTTGCCGTAAAGACAAC | 6 |
| Fru_rev2 | GGTATATCTCTTTACATAAAGTTAACCGCTTTAATATATC | 7 |

Variants were cloned to the expression vector, a pUB110 derivate, by using N EBuilderOHifi DNA Assembly Master Mix (NEB, Frankfurt) with the vector - insert ratio of 1:3. A schematic picture of the expression plasmids is presented in Figure 2. For construction purposes, expression plasmids were transformed by induced competence into *Bacillus subtilis* SCK6 as described in Zhang and Zhang 2011. The transformed cells were plated onto LB (Luria-Bertani) plates supplemented with 10 mg/L Kanamycin. Plates were incubated for 20 h at 37 °C. Arising colonies were picked and plasmid was isolated using QiaPrep MiniPrep Kit (Qiagen, Hilden). Inserts were verified by sequencing. The expression plasmids were transformed in *Bacillus subtilis* host cells via induced competence. The constructed cell strains are listed in Table 2.

**Table 2.** Cell strains constructed for expression of wild-type LFRU fructanase and its truncated variant.

| Name | Molecule | Description |
|---|---|---|
| Host | - | Host cell strain *Bacillus subtilis* |
| Bs TR | Truncated | The truncated LFRU variant (S-layer domains removed) according to SEQ ID NO: 2 |
| Bs WT | Wild type | The full-length wild-type LFRU fructanase according to SEQ ID NO: 1 |

The three cell strains were inoculated in a standard expression medium in deep-well plates under protein inducing conditions and incubated for 30 h at 37 °C. Cell culture supernatants were harvested and used for SDS-PAGE and fructanase activity analyses. As shown in the SDS-PAGE gel in Figure 3 lanes 6-9, only the truncated fructanase variant having the S-layer domains deleted, was produced in *Bacillus subtilis* as intact (-130 kDa) enzyme having fructanase activity.

**Example 3. Activity analysis of fructanase variants produced in *Bacillus subtilis***

The fructanase enzyme activity of the fructanase variants was measured from the culture supernatants as the release of reducing sugars (i.e. fructose residues) from inulin using Fluent® automation workstation (Tecan Group Ltd, Männedorf, Switzerland).

The samples were diluted in a 0.05 M sodium acetate buffer pH 5.0, and the substrate, inulin from chicory (Sigma No. I2255), was dissolved in the same buffer. A substrate concentration of 0.25 % (w/v) was used. Sample dilution (25 $\mu$L) was mixed with the preheated substrate (225 $\mu$L) in the DeepWell plate and incubated at 37 °C, for 15 min. After 15 min incubation, 375 $\mu$L dinitrosalicylic reagent (10 g of 3,5-dinitrosalicylic acid, 16 g of NaOH, 300 g of potassium sodium tartrate tetrahydrate dissolved in distilled water and filled-in to 1 liter) was added, and the reaction was stopped by boiling the mixture for 8 min. Samples were cooled for 5 min at 4 °C and thereafter for 3 min at 20 °C. After the cooling step 200 $\mu$L of each reaction mixture was transferred into a 96 well microplate. The absorbance was measured at 540 nm in a microplate spectrophotometer (Tecan, Spark).

A calibration curve was obtained using fructose as the standard (7.5, 10, 14, 20, 30, 40 and 50 $\mu$mol/mL). Fructose standards were treated identically to the samples. One enzymatic unit was defined as an amount of the enzyme which produces 1 $\mu$mol of reducing sugars per mL. The amount of released fructose is calculated from the calibration curve.

The fructanase activity results from samples obtained from the empty host strain cells and strains expressing the wild type LFRU fructanase or the truncated variant fructanase are shown in Table 3, the fructanase activity being indicated as units per ml (U/ml). Each sample type was measured in four parallel samples. The measured culture supernatants show only fructanase activity for the samples collected from the strain expressing the truncated LFRU variant (SEQ ID NO: 2). The samples collected from the empty host strain and the strain expressing full-length wild-type LFRU fructanase (SEQ ID NO: 1) do not show any fructanase activity.

**Table 3.** Fructanase activity of the supernatants collected from empty host cell strain (-), the strain expressing wild-type LFRU fructanase (SEQ ID NO: 1) and the strain expressing truncated LFRU variant (SEQ ID NO: 2).

| Molecule | Fructanase activity (U/ml) |
|---|---|
| - | 0 |
| - | 0 |
| - | 0 |
| - | 0 |
| SEQ ID NO: 2 | 17 |
| SEQ ID NO: 2 | 15 |
| SEQ ID NO: 2 | 18 |
| SEQ ID NO: 2 | 15 |
| SEQ ID NO: 1 | 0 |
| SEQ ID NO: 1 | 0 |
| SEQ ID NO: 1 | 0 |
| SEQ ID NO: 1 | 0 |

Various embodiments have been presented. It should be appreciated that in this document, words comprise, include, and contain are each used as open-ended expressions with no intended exclusivity.

The foregoing description has provided by way of non-limiting examples of particular implementations and embodiments a full and informative description of the best mode presently contemplated by the inventors for carrying out the invention. It is however clear to a person skilled in the art that the invention is not restricted to details of the embodiments presented in the foregoing, but that it can be implemented in other embodiments using equivalent means or in different combinations of embodiments without deviating from the characteristics of the invention.

Furthermore, some of the features of the afore-disclosed example embodiments may be used to advantage without the corresponding use of other features. Consequently, any appropriate combination of the embodiments and the aspects may be formed. Any combination of aspects or embodiments as disclosed herein may also be made without at least one non-essential feature disclosed in an aspect or embodiment. As such, the foregoing description shall be considered as merely illustrative of the principles of the present invention, and not in limitation thereof. Hence, the scope of the invention is only restricted by the appended patent claims.

**References**

Mirdita M, Schütze K, Moriwaki Y, Heo L, Ovchinnikov S, Steinegger M. (2022) ColabFold: Making protein folding accessible to all. Nature Methods, 19, 679-682.

Sambrook and Russell (2001) Molecular cloning, a laboratory manual. Cold Spring Harbor Laboratory, New York, US.

Zhang and Zhang (2011) Simple, fast and high-efficiency transformation system for directed evolution of cellulase in Bacillus subtilis. Microbial Biotechnology 4(1), 98-105.

**Claims**

1.    A variant polypeptide having:

fructanase activity;

an amino acid sequence having at least 80 % amino acid sequence identity with the amino acids 383 - 858 of SEQ ID NO: 1; and

a size of 476 - 1165 amino acids;

wherein the amino acid numbering of the variant polypeptide corresponds to the amino acid numbering of the SEQ ID NO: 1.

2. The variant polypeptide of claim 1, having amino acids corresponding to amino acids 1 - 1165 of the SEQ ID NO: 1 or less, preferably the variant polypeptide has amino acids corresponding to amino acids 1 - 858 of the SEQ ID NO: 1 or less, more preferably the variant polypeptide has amino acids corresponding to amino acids 383 - 858 of the SEQ ID NO: 1.

3. The variant polypeptide of claim 1 or 2, having amino acids corresponding to amino acids:

383 - 858, or 383 - 953, or 383 - 1118, or 383 - 1165, or
278 - 858, or 278 - 953, or 278 - 1118, or 278 - 1165, or
96 - 858, or 96 - 953, or 96 - 1118, or 96 - 1165, or
1 - 858, or 1 - 953, or 1 - 1118, or 1 - 1165;

of the SEQ ID NO: 1 and wherein the amino acids have at least 80 % amino acid sequence identity with the corresponding amino acids of the SEQ ID NO: 1.

4. The variant polypeptide of any preceding claim, having at least 85 %, preferably at least 90 %, more preferably at least 95 %, even more preferably at least 98 % amino acid sequence identity with the amino acids 1 - 1165 of the SEQ ID NO: 1.

5. The variant polypeptide of any preceding claim, having at least 100 or at least 150 amino acids deleted from the amino acids corresponding to the amino acids 859 - 1279 of the SEQ ID NO: 1, preferably having the at least 100 amino acids deleted from the amino acids corresponding to the amino acid 1166 - 1279 of the SEQ ID NO: 1.

6. The variant polypeptide of any preceding claim, having at least 100 or at least 150 amino acids deleted from the amino acids corresponding to the amino acids 1 - 382 of the SEQ ID NO: 1.

7. The variant polypeptide of any preceding claim, obtained via inactivation of at least one SLAP domain, or via deletion or substitution of amino acids of at least one SLAP domain of a fructanase enzyme having a sequence comprising at least one SLAP domain, the variant polypeptide thereby having at least one non-functional SLAP domain and/or at least one SLAP domain deleted.

8. The variant polypeptide of any preceding claim, having an increased fructanase productivity and/or improved fructan degrading performance, when compared to a polypeptide having the sequence SEQ ID NO: 1.

9. A fusion protein, comprising the variant polypeptide according to any one of claims 1-8, and at least one:

an amino acid sequence providing a secretory signal sequence;
an amino acid sequence which facilitates purification, such as an affinity tag or His-tag;
an amino acid sequence controlling and/or enhancing the production of the fusion protein, such as a carrier polypeptide;
a linker sequence;
an amino acid sequence encoding a protease cleavage site;
an amino acid sequence having an enzyme activity; and/ or
an amino acid sequence providing for the fusion protein with binding affinity, such as a carbohydrate binding moiety.

10. An enzyme composition comprising the variant polypeptide of any one of claims 1-8 or the fusion protein of claim 9.

11. A recombinant host cell comprising genetic elements that allow producing at least one variant polypeptide of any one of claims 1-8, or the fusion protein of claim 9.

12. A method of manufacturing the variant polypeptide of any one of claims 1-8, or the fusion protein of claim 9, comprising:

cultivating the recombinant host cell of claim 11 in conditions allowing production of the at least one variant polypeptide of any one of claims 1-8, or the fusion protein of claim 9; and
optionally recovering the variant polypeptide or the fusion protein.

13. Use of the variant polypeptide of any one of claims 1-8, and/or the fusion protein of claim 9 and/or the enzyme composition of claim 10, for degradation of fructan in plant-based material.

14. A premix for baking, comprising the variant polypeptide of any one of claims 1-8, and/or the fusion protein of claim 9 and/or the enzyme composition of claim 10.

15. A variant polypeptide having:

fructanase activity;
an amino acid sequence having at least 80 % amino acid sequence identity with the amino acids 1-1165 of SEQ ID NO: 1; and
at least one SLAP domain deleted or at least one non- functional SLAP domain, obtained via deletion or substitution of amino acids,

wherein the amino acid numbering of the variant polypeptide corresponds to the amino acid numbering of the SEQ ID NO: 1.

## Fig. 1

**Fig. 2**

**Fig. 3**

**EP 4 512 895 A1**

Europäisches Patentamt
European Patent Office
Office européen des brevets

# EUROPEAN SEARCH REPORT

Application Number

EP 23 19 2550

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/020665 A2 (KIWI HEALTH INC [US]) 27 January 2022 (2022-01-27) * sequence 7 * | 1-4,9-14 | INV. C12N9/42 |
| X | WO 2017/220864 A1 (FAZER AB OY KARL [FI]) 28 December 2017 (2017-12-28) | 1-4,9-14 | |
| Y | * the whole document * | 5-8,15 | |
| A | KLOTZ COURTNEY ET AL: "Deletion of S-Layer Associated Ig-Like Domain Protein Disrupts the Lactobacillus acidophilus Cell Surface", FRONTIERS IN MICROBIOLOGY, vol. 11, 17 March 2020 (2020-03-17), XP93109776, Lausanne ISSN: 1664-302X, DOI: 10.3389/fmicb.2020.00345 * the whole document * | 1-15 | |
| Y | Li Qing: "Characterization of lifestyle-associated metabolic traits of host-adapted lactobacilli that are relevant in food fermentation", Doctoral Thesis, 31 December 2020 (2020-12-31), XP93109778, Retrieved from the Internet: URL:https://era.library.ualberta.ca/items/d5f246a0-b849-4df7-ad5d-a50fe14f5cae/view/d05b0960-7f06-4338-892c-6454f0a6fd1e/Li_Qing_202009_PhD.pdf [retrieved on 2023-12-06] * the whole document * * In particular second paragraph on page 104 * | 5-8,15 | TECHNICAL FIELDS SEARCHED (IPC) C12N |

The present search report has been drawn up for all claims

| Place of search | Date of completion of the search | Examiner |
|---|---|---|
| Munich | 7 December 2023 | Strobel, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

27

## ANNEX TO THE EUROPEAN SEARCH REPORT
## ON EUROPEAN PATENT APPLICATION NO.

**EP 23 19 2550**

This annex lists the patent family members relating to the patent documents cited in the above-mentioned European search report.
The members are as contained in the European Patent Office EDP file on
The European Patent Office is in no way liable for these particulars which are merely given for the purpose of information.

**07-12-2023**

| Patent document cited in search report | | Publication date | Patent family member(s) | | | Publication date |
|---|---|---|---|---|---|---|
| WO 2022020665 | A2 | 27-01-2022 | AU | 2021310923 | A1 | 09-02-2023 |
| | | | CA | 3186646 | A1 | 27-01-2022 |
| | | | EP | 4185691 | A2 | 31-05-2023 |
| | | | US | 2023303993 | A1 | 28-09-2023 |
| | | | WO | 2022020665 | A2 | 27-01-2022 |
| WO 2017220864 | A1 | 28-12-2017 | AU | 2017281684 | A1 | 06-12-2018 |
| | | | CA | 3023665 | A1 | 28-12-2017 |
| | | | DK | 3475419 | T3 | 18-10-2021 |
| | | | EP | 3475419 | A1 | 01-05-2019 |
| | | | ES | 2896774 | T3 | 25-02-2022 |
| | | | FI | 127298 | B | 15-03-2018 |
| | | | NZ | 748482 | A | 26-08-2022 |
| | | | RU | 2018136950 | A | 23-07-2020 |
| | | | US | 2019174773 | A1 | 13-06-2019 |
| | | | US | 2020296976 | A1 | 24-09-2020 |
| | | | WO | 2017220864 | A1 | 28-12-2017 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017220864 A

**Non-patent literature cited in the description**

- **MIRDITA M** ; **SCHÜTZE K** ; **MORIWAKI Y** ; **HEO L** ; **OVCHINNIKOV S** ; **STEINEGGER M**. ColabFold: Making protein folding accessible to all. *Nature Methods*, 2022, vol. 19, 679-682
- **SAMBROOK** ; **RUSSELL**. Molecular cloning, a laboratory manual. Cold Spring Harbor Laboratory, 2001
- **ZHANG** ; **ZHANG**. Simple, fast and high-efficiency transformation system for directed evolution of cellulase in Bacillus subtilis. *Microbial Biotechnology*, 2011, vol. 4 (1), 98-105